# EUROPEAN PATENT SPECIFICATION

(11) **EP 0 833 847 B1**
(45) Date of publication and mention of the grant of the patent: **24.09.2003**
(21) Application number: 96922527.5
(22) Date of filing: 21.06.1996
(51) Int. Cl.: C07K 14/725, C07K 14/525, C07K 1/00, G01N 33/68

(54) **CRYSTALS OF FRAGMENTS OF CD40 LIGAND AND THEIR USE**
KRISTALLE VON FRAGMENTEN VON CD40-LIGANDEN UND DEREN VERWENDUNG
CRISTAUX DE FRAGMENTS DU LIGAND CD40 ET LEUR UTILISATION

(30) Priority: 22.06.1995 US 448 P
(43) Date of publication of application: 08.04.1998
(73) Proprietor: BIOGEN, INC., Cambridge Massachusetts 02142 (US)
(72) Inventor: KARPUSAS, Mihail N., Roslindale, MA 02131 (US); SINGH, Juswinder, Malden, MA 02148 (US); THOMAS, David W., Wellesley, MA 02181 (US)
(74) Representative: VOSSIUS & PARTNER
(86) International application number: US9610664
(87) International publication number: WO97000895

(56) References cited:
- EP-A- 0 555 880
- EP-A- 0 585 943
- WO-A-93/08207
- WO-A-94/17196
- JOURNAL OF IMMUNOLOGICAL METHODS, vol. 149, no. 2, 15 June 1992, NEW YORK US, pages 655-660, XP002017167 W C FANSLOW ET AL.: "Soluble forms of CD40 inhibit biologic responses of human B cells"
- CHEMICAL ABSTRACTS, vol. 120, no. 3, 17 January 1994 Columbus, Ohio, US; abstract no. 28892n, M C PEITSCH & V C JONGENEEL: "A 3-D model for the CD40 ligand predicts that it is a compact trimer similar to the tumor necrosis factor" page 659; XP002017169 & INT. IMMUNOL. , vol. 5, no. 3, 1993, pages 233-238,
- BIOCHEMISTRY, vol. 34, no. 31, 8 August 1995, EASTON, PA US, pages 9884-9892, XP002017168 J BAJORATH ET AL.: "Analysis of gp39/CD40 interactions using molecular models and site-directed mutagenesis"
- CHEMICAL ABSTRACTS, vol. 123, no. 23, 4 December 1995 Columbus, Ohio, US; abstract no. 311922s, M KARPUSAS ET AL.: "2A crystal structure of an extracellular fragment of human CD40 ligand" page 700; XP002017170 & STRUCTURE, vol. 3, no. 10, 1995, LONDON, pages 1031-1039,

## Description

The present invention relates to a crystalline form of a soluble fragment of the extracellular domain of human CD40 ligand, and more particularly, to a crystal of a CD40L fragment containing residues Gly116 to Leu261 ("sCD40L(116-261)") and its structure, obtained by x-ray diffraction. In addition, this invention relates to methods of using a crystal structure of CD40L, or a fragment thereof, and, specifically sCD40L(116-261), to design, screen, and optimize compounds that affect CD40L activity.

### BACKGROUND OF THE INVENTION

The immune system consists of many complex processes, which, together, protect the body against toxic and/or pathogenic agents. Generally, an immune response is initiated when certain cells within the body recognize the toxic or pathogenic agents. The response is then communicated through any of a series of means, such as cell-cell contact or soluble mediators, to other cells involved in the immune response.

CD40 ligand ("CD40L") has been shown to mediate functional T and B cell interactions involved in the immune response (EP-A-585 943). The term CD40L refers to a genus of polypeptides which are capable of binding CD40, or homologs thereof. In fact, the interaction of CD40L with CD40 expressed by B-cells is the principal molecular interaction responsible for T cell contact dependent induction of B cell growth and differentiation, to antigen specific antibody production. Binding of T-cell CD40L to its counter-receptor CD40 expressed on the surface of B lymphocytes results in pleiotropic activities, including activation of antibody isotype switching, prevention of B cell apoptosis, establishment of immunological memory, germinal center formation in lymphoid tissues, modulation of cytokine production in activated B cells and B cell proliferation and differentiation

A soluble version of CD40L can be made from the extracellular region or fragments thereof. As used herein, the term CD40L includes soluble CD40L polypeptides lacking transmembrane and intracellular regions, homologs and analogs of CD40L or derivatives thereof. Early studies have shown that CD40L is a member of the tumor necrosis factor (TNF) family of cytokines. Other members of this family include TNF-α, LT-α (lymphotoxin-α, known also as TNF-β), LT-β, Fas ligand, CD30L and CD27L. CD40L is a membrane bound polypeptide with an extracellular region at its C terminus, a transmembrane region, and an intracellular region at the N terminus.

Several mutations of CD40L are known to cause an X-chromosome-linked severe immunodeficiency, known as Hyper-IgM syndrome ("HIGMS"). HIGMS is characterized by normal or elevated levels of IgM, but absent or low levels of IgG, IgA and igE in serum. A murine CD40L gene "knockout" also lacks expression of IgG, IgA and IgE, similar to Hyper-IgM syndrome in man. These observations suggest that CD40 signaling is required for IgG, IgA, and IgE production, and that this function is non-redundant with other signaling/adhesion pathways. Studies show that CD40L may also play roles in certain diseases such as arthritis, lupus, multiple sclerosis, graft versus host disease, ulcerative colitis, allergies, tissue transplantation and generation of antibodies against antigenic drugs. Thus, it is apparent that interfering with the CD40-CD40L interaction has wide reaching therapeutic and diagnostic applications.

There is presently an interest in designing, identifying or obtaining potential drug candidates which would interfere with the CD40-CD40L interaction. The recent emergence of drug design to identify candidates that play a role in a physiologically relevant biological pathway has provided a useful approach for obtaining, or designing, lead compounds for drugs.

Generally, this approach requires selecting a protein target molecule which plays a role in a physiologically relevant biological pathway. Typically, once a ligand, natural or synthesized, is found for the target molecule, it is modified or optimized to produce a candidate with the desired properties.

In order to more efficiently design or modify a ligand, it is useful to have a three-dimensional structure for the bioactive conformation of a known ligand as it binds to the target protein molecule. Furthermore, it is valuable to understand the detailed interactions of the ligand with its target protein by examining the three-dimensional structure of the protein target in complex with its known ligand. This allows the artisan to preserve the critical interactions with the protein, while modifying candidate ligands to interact more precisely with the protein, resulting in better potency and specificity.

However, the three dimensional crystal structure of the protein target is frequently unavailable due to the significant effort required to obtain crystals of sufficient size and resolution to provide accurate information regarding the structure. For example, it is time consuming and often difficult to express, purify and characterize a protein. Additionally, once the protein of sufficient purity is obtained, it must be crystallized to a size and clarity which is useful for x-ray diffraction and subsequent structure solution. Thus, although crystal structures can provide a wealth of valuable information in the field of drug design and discovery, crystals of certain biologically relevant compounds such as CD40L, are not readily available to those skilled in the art.

Furthermore, although the amino acid sequence of a target protein or its ligand, such as CD40L, is known, this sequence information does not allow an accurate prediction of the crystal structure of the protein/ligand. Nor does the sequence information afford an understanding of the structural, conformational and chemical interactions between a ligand such as CD40L and its protein target.

Thus, there is a need for a detailed knowledge of the crystalline three-dimensional structure of the extracellular domain of CD40L, to effectively design, screen or optimize compounds capable of interfering with the CD40L -CD40 interactions. Although the binding of CD40 and its ligands has been studied, and similarities found between this system and the TNF system, the differences that exist suggest that these two ligand-receptor systems utilize spatially overlapping, but nonidentical and nonconserved sites of contact residues with different molecular determinants of binding.

Crystals of CD40L or fragments of CD40L of a size and quality such as to allow performance of x-ray diffraction studies would enable those skilled in the art to conduct studies relating to the binding properties of CD40L, as well as the binding properties of molecules or molecular complexes which may associate with CD40L or a fragment thereof.

### SUMMARY OF THE INVENTION

Accordingly, the present invention is directed to crystals of CD40L or crystals of fragments of CD40L, of sufficient size and quality to obtain useful information about the properties of CD40L and molecules or complexes which may associate with CD40L or CD40. The claimed invention provides the three-dimensional crystal structure of the Gly116 to Leu261 fragment of CD40L, which can be used to identify binding sites to solve the structure of unknown crystals, to provide mutants having desirable binding properties, and ultimately, to design, characterize, or identify molecules or chemical entities capable of interfering with the interaction between CD40 and CD40L.

Additional features and advantages of the invention will be set forth in the description which follows, and in part will be apparent from the description, or may be learned by practice of the invention. The objectives and other advantages of the invention will be realized and attained by the compositions and methods particularly pointed out in the written description and claims hereof, as well as in the appended drawings.

To achieve these and other advantages, and in accordance with the purpose of the invention, as embodied and broadly described herein, the invention relates to a crystal of CD40L. More particularly, the invention relates to a crystal formed by a functional fragment of the extracellular domain of sCD40L(116-261), wherein the crystal has cell constants a=b=77.17 x 10⁻¹⁰ M (Å), c= 90.46 x 10⁻¹⁰ M (Å), α = β =90°, γ = 120°, and a space group of R3, and equivalents of that crystal. The claimed crystals of CD40L are substantially described by the structural coordinates identified in Table 1. The claimed crystals in certain embodiments are characterized by a binding site moiety comprising Ile127, Ser128, Glu129, Ala130, Ser131, Thr135, Ser136, Ala141, Glu142, Lys143, Gly144, Tyr145, Tyr146, Cys178, Asn180, Ser185, Gln186, Ala187, Pro188, Ile190, Ala191, Ser192, Ser197, Pro198, Gly199, Arg200, Phe201, Glu202, Arg203, Ile204, Arg207, Ala209, Thr211, Pro217, Cys218, Gly219, Gln220, Glu230, Leu231, Gln232, Asn240, Val241, Thr242, Asp243, Ser245, Val247, Ser248, His249, Gly250, Thr251, Gly252 and Phe253.

Additionally, the invention relates to crystals of molecules having a binding site comprising at least Arg207, and preferably, amino acids Lys143, Arg203, Arg207 and Tyr145. The claimed crystals in certain embodiments, are formed from molecules having a binding site moiety comprising Arg207 surrounded by at least two hydrophobic residues. Mutants, homologs, co-complexes and fragments of the claimed crystals are also contemplated herein.

The claimed invention in certain embodiments relates to heavy atom derivatives of the crystallized form of a sCD40L(116-261), and, more specifically, the heavy atom derivatives of the crystallized form of CD40L described above. In various embodiments, the claimed invention relates to methods of preparing crystalline forms of CD40L, or fragments thereof, by providing an aqueous solution comprising at least a fragment of CD40L, providing a reservoir solution comprising a precipitating agent, mixing a volume of the CD40L solution with a volume of the reservoir solution and crystallizing the resultant mixed volume. In certain embodiments, the crystal is derived from an aqueous solution comprising sCD40L(116-261). In various embodiments, the concentration of CD40L in the aqueous solution is about 1 to about 50 mg/ml, preferably about 5 mg/ml to about 15 mg/ml, and most preferably, about 10 mg/ml. The precipitating agents used in the invention may be any precipitating agent known in the art, preferably one selected from the group consisting of sodium citrate, ammonium sulfate and polyethylene glycol. Any concentration of precipitating agent may be used in the reservoir solution, however it is preferred that the concentration be about 1.0 M to about 1.5 M, more preferably about 1.2 M. Similarly, the pH of the reservoir solution may be varied, preferably between about 4 to about 10, most preferably about 7.5.

Various methods of crystallization can be used in the claimed invention, including, but not limited to, vapor diffusion, batch, liquid bridge, or dialysis. Vapor diffusion crystallization is preferred.

Additionally, the claimed invention relates to methods of using the claimed crystal, and the structure coordinates in methods for screening, designing, or optimizing molecules or other chemical entities that may interfere with the interaction between CD40 and CD40L. Thus, the structural coordinates of CD40L or portions thereof can be used to solve the crystal structure of a mutant, homologue or co-complex of CD40L or a fragment thereof, as well as to solve other unknown crystals which asociate with CD40L or fragments thereof.

In some embodiments, the structural coordinates of the CD40L can be used to evaluate a chemical entity to obtain information about the binding of the chemical entity to CD40L. The claimed invention also relates to the structural coordinates described in Table 1. The structural coordinates can be used to characterize chemical entities which interfere with the relationship between CD40 or CD40L such as inhibitors or agonists. The coordinates can also be used to optimize binding characteristics, to determine the orientation of ligands in the binding site of CD40L or CD40. One skilled in the art will appreciate the numerous uses of the claimed invention in the areas of drug design, screening and optimization of drug candidates, as well as in determining additional unknown crystal structures.

In various embodiments, the claimed invention relates to a machine readable data storage medium having a data storage material encoded with machine readable data, which, when read by an appropriate machine, can display a three dimensional representation of a crystal. The crystals displayed comprise a fragment of CD40L such as that described by the coordinates in Table 1, or a crystal having a binding site comprising amino acids Lys143, Arg203, Arg207 and Tyr145.

In other embodiments, the claimed invention relates to a method for determining a at least a portion of a three dimensional structure of a chemical entity or molecular complex by calculating phases from the structural coordinates of a crystal of a fragment of CD40 ligand, calculating the electron density map from the phases obtained, and then determining at least a portion of the unknown structure based upon the electron density map.

In yet other embodiments, the invention relates to methods for evaluating the ability of a chemical entity to associate with CD40 or CD40L. The methods employ computational or experimental means to perform a fitting operation between the chemical entity and the CD40 or CD40L to obtain data related to the association, and analyzing the data to determine the characteristics. Chemical entities identified by these methods which are capable of interfering with the in vivo or in vitro association between CD40 and CD40 L are also encompassed by the claimed invention. The claimed chemical entities may comprise binding sites substantially similar to those of CD40L, or, alternatively may comprise binding sites capable of associating with the binding sites of CD40L.

It is to be understood that both the foregoing general description and the following detailed description are exemplary and explanatory and are intended to provide further explanation of the invention as claimed.

The accompanying drawings are included to provide a further understanding of the invention and are incorporated in and constitute a part of this specification, illustrate several embodiments of the invention, and together with the description, serve to explain the principles of the invention.

### BRIEF DESCRIPTION OF THE FIGURES

Figure 1 is a depiction of the representative regions of a 2Fₒ-F_{c} electron density map contoured at 2.5σ showing residues in the vicinity of the CD40 binding site.
Figure 2 is an electron density map showing a view of a network of 3 tyrosine and 3 histidine residues formed in the vicinity of the center of the trimer of CD40.
Figure 3 is a stereo drawing of the 3-dimensional structure of human CD40L specifically, CD40L Cα backbone.

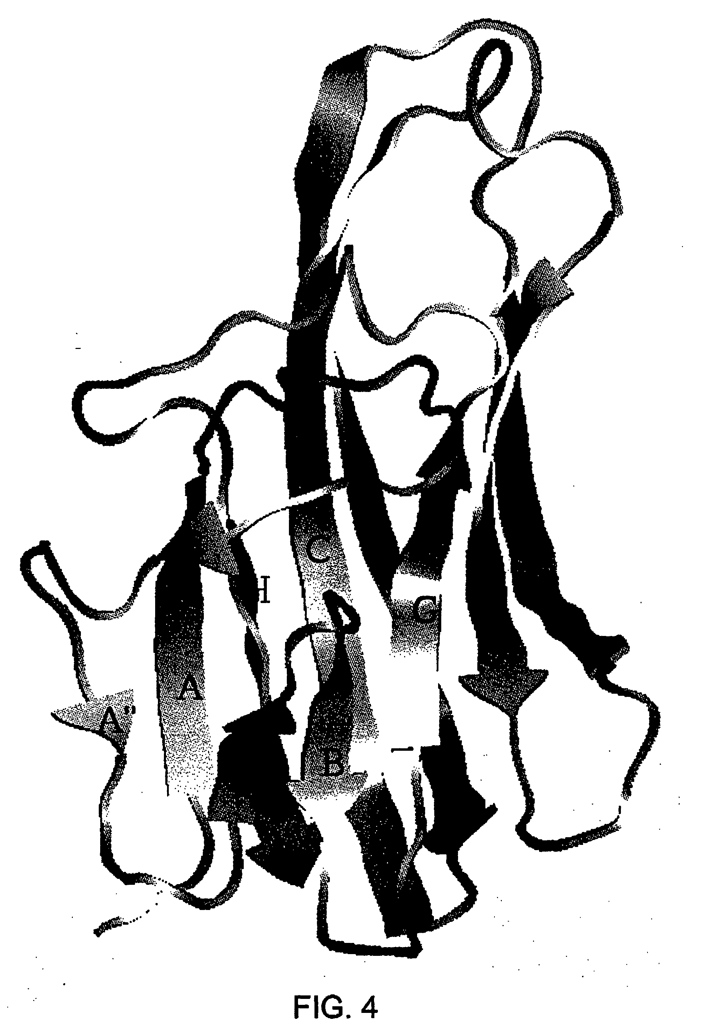

Figure 4 is a ribbon representation and secondary structure assignment of CD40L.
Figure 5 is a sequence alignment of the TNF-like domains of TNFα, Ltα and CD40L based on structural considerations.
Figure 6 is a graph of temperature factors of main chain atoms as a function of residue number. Residues are numbered with Pro120 as residue 1.
Figure 7 is a depiction of residues involved in Hyper-IgM and designed mutations. The third monomer has been removed for clarity.
Figure 8 is a summary of sCD40L(116-261) crystallographic and refinement data.

### DETAILED DESCRIPTION OF THE INVENTION

In order that the invention described herein may be more fully understood, the following detailed description is set forth.

The present invention relates to a crystal of a soluble fragment of the extracellular domain of the CD40 ligand. Specifically, it relates to a crystal of a protein comprising the sequence from Gly116 to Leu261 (sCD40L(116-261)), the structure of sCD40L(116-261) as determined by X-ray crystallography, and the use of the sCD40L(116-261) structure and that of its homologs, mutants and co-complexes to design, identify, characterize, screen and/or optimize candidate inhibitors or agonists of CD40L activity.

### A. DEFINITIONS

The term CD40 ligand ("CD40L") as used herein refers to a genus of polypeptides which are capable of binding to CD40, or homologs or fragments thereof. The term as used herein includes sCD40L(116-261), homologs, mutants, equivalents and fragments thereof.

The term "co-complex" refers to a CD40L or a mutant or homolog of CD40L in covalent or non-covalent association with a chemical entity.

The term "homolog" refers to a protein having at least about 50% amino acid sequence identity with the protein to which it is being compared.

The term "mutant" refers to a CD40L or fragment thereof, characterized by the replacement, deletion, or insertion of at least one amino acid from the wild-type. Such a mutant may be prepared, for example, by expression of a CD40L previously altered in its coding sequence by oligonucleotide-directed mutagenesis.

The term " positively charged amino acid" includes any amino acid, natural or unnatural, having a positively charged side chain under normal physiological conditions. Examples of positively charged naturally occurring amino acids are arginine, lysine and histidine.

The term "negatively charged amino acid" includes any amino acid, natural or unnatural, having a negatively charged side chain under normal physiological conditions. Examples of negatively charged naturally occurring amino acids are aspartic acid and glutamic acid.

The term "hydrophobic amino acid" means any amino acid having an uncharged, nonpolar side chain that is relatively insoluble in water. Examples are alanine, leucine, isoleucine, valine, proline, phenylalanine, tryptophane and methionine.

The term "hydrophilic amino acid" means any amino acid having an uncharged, polar side chain that is relatively soluble in water. Examples are serine, threonine, tyrosine, asparagine, glutamine, and cysteine.

The term "altered surface charge" means a change in one or more of the charge units of a mutant polypeptide, at physiological pH, as compared to CD40 ligand. The change in surface charge can be determined by measuring the isoelectric point (pI) of the polypeptide molecule containing the substituted amino acid and comparing it to the pI of the wild-type molecule.

The term "associating with" refers to a condition of proximity between two chemical entities, or portions thereof, for example, a CD40 ligand or portions thereof and a chemical entity. The association may be non-covalent, wherein the juxtaposition is energetically favored by hydrogen bonding, van der Waals interaction, or electrostatic interaction, or it may be a covalent association.

The term "binding site" refers to any or all of the sites where a chemical entity binds or associates with another entity.

The term "structural coordinates" refers to the coordinates derived from mathematical equations related to the patterns obtained on diffraction of a monochromatic beam of X-rays by the atoms (scattering centers) of molecule in crystal form. The diffraction data are used to calculate an electron density map of the repeating units of the crystal. Those skilled in the art will understand that the data obtained are dependent upon the particular system used, and hence, different coordinates may in fact describe the same crystal if such coordinates define substantially the same relationship as those described herein. The electron density maps are used to establish the positions of the individual atoms within the unit cell of the crystal.

Those of skill in the art understand that a set of structural coordinates determined by X-ray crystallography is not without standard error. Table 1 is the atomic coordinates of sCD40L(116-261). For the purpose of this invention, any set of structural coordinates of CD40L(116-261) that have a root mean square deviation of equivalent protein backbone atoms of less than about 2 x 10⁻¹⁰ M (Å) when superimposed--- using backbone atoms-- on the structural coordinates in Table 1 shall be considered identical. Preferably the deviation is less than about 1 x 10⁻¹⁰ M (Å) and more preferably less than about 0-5 x 10⁻¹⁰ M (Å).

The term "heavy atom derivatization" refers to a method of producing a chemically modified form of a crystallized CD40 ligand. In practice, a crystal is soaked in a solution containing heavy metal atom salts, or organometallic compounds, e.g., lead chloride, gold thiomalate, thimerosal or uranyl acetate, which can diffuse through the crystal and bind to the surface of the protein. The location of the bound heavy metal atom(s) can be determined by X-ray diffraction analysis of the soaked crystal. This information can be used to generate the phase information used to construct the three-dimensional structure of the molecule.

The term "unit cell" refers to a basic shaped block. The entire volume of a crystal may be constructed by regular assembly of such blocks. Each unit cell comprises a complete representation of the unit of pattern, the repetition of which builds up the crystal.

The term "space group" refers to the arrangement of symmetry elements of a crystal.

The term "molecular replacement" refers to a method that involves generating a preliminary structural model of a crystal whose structural coordinates are unknown, by orienting and positioning a molecule whose structural coordinates are known e.g. the CD40 ligand coordinates in Table 1, within the unit cell of the unknown crystal, so as to best account for the observed diffraction pattern of the unknown crystal. Phases can then be calculated from this model, and combined with the observed amplitudes to give an approximate Fourier synthesis of the structure whose coordinates are unknown. This in turn can be subject to any of the several forms of refinement to provide a final accurate structure of the unknown crystal. (See, e.g., Lattman, E., "Use of the Rotation and Translation Functions", Methods in Enzymology, 115, pp. 55-77 (1985); Rossman, ed., "The Molecular Replacement Method", Int. Sci. Rev. Ser. No. 13, Gordon and Breach, New York (1972). Specifically incorporated by reference herein.) Using the structural coordinates of CD40 ligand provided by this invention, molecular replacement may be used to determine the structural coordinates of a crystalline co-complex, unknown ligand, mutant, homolog, or of a different crystalline form of CD40 ligand. Additionally, the claimed crystal and its coordinates may be used to determine the structural coordinates of a chemical entity which associates with CD40L or CD40.

The term "chemical entity" as used herein shall mean, for example, any molecule, molecular complex, compound or fragment thereof.

CD40L mutants may be generated by site-specific incorporation of natural or unnatural amino acids into CD40L using general biosynthetic methods known to those skilled in the art. For example, the codon encoding the amino acid of interest in wild-type CD40L may be replaced by a "blank" nonsense codon, such as TAG, using oligonucleotide-directed mutagenesis. A suppressor tRNA directed against this codon can then be chemically aminoacylated in vitro with the desired amino acid. The aminoacylated tRNA can then be added to an in vitro translation system to yield a mutant sCD40L with the site-specific incorporated amino acid.

The term soluble fragment of CD40L and any equivalent term used herein, refers to a functional fragment of CD40L. The term "functional" as used in this context refers to a soluble fragment of the extracellular domain that is capable of binding to, or associating with, a CD40, CD40-Ig fusion protein or any fragments or homologs thereof, including molecular complexes comprising fragments thereof. Such binding may be demonstrated through immunoprecipitation experiments, using standard protocols known in the art.

### A. CD40L, its Crystal, and its Biological Implications

It will be understood that throughout the specification and claims, the positional location of the amino acids described is not an absolute value, but rather, defines the relative relationship of the residues. Thus it is intended that the present invention encompass the sequences having the same or similar relative positions.

For the first time, the present invention permits the use of molecular design techniques to design, screen and optimize chemical entities and compounds, including inhibitory compounds, capable of binding to the active site or accessory binding site of CD40L, in whole or in part. CD40 ligand (CD40L) is a T cell membrane-bound pleiotropic cytokine of considerable biomedical interest because of its involvement in important immune system functions mediated by the binding of CD40L to CD40. CD40 is a molecule expressed on the surface of B cells and other cell types. CD40L is found in various organisms, such as humans, mice, rats, pigs, etc. The claimed invention is not intended to be limited to any particular species or organism.

CD40L is a member of the tumor necrosis factor family of ligands. The crystal structure of another member of this family, lymphotoxin-α (LTα) complexed with its receptor has been described and has be used as a framework for understanding the CD40L crystal structure. However, despite certain similarities, the differences between the LT system and the CD40 system confirm that the two ligand-receptor systems utilize spatially overlapping, but nonidentical and nonconserved sites of contact residues with different molecular determinants of binding.

Considering the complexity and overlap of the various immune system processes, the fact that CD40 signaling appears to be non-redundant suggests that inhibiting CD40L signaling may have important therapeutic applications. The crystal structure of CD40L presented here is expected to be useful in the design, identification, characterization and optimization of such therapeutic agents.

The following detailed description of applicants invention encompasses the (a) crystal structure of CD40L(116-261) and the coordinates thereof, (b) the binding sites of CD40L, (c) methods of making a CD40L crystal, and (d) methods of using the CD40L crystal and its structural coordinates.

### (a.) Crystal Structure of CD40 Ligand

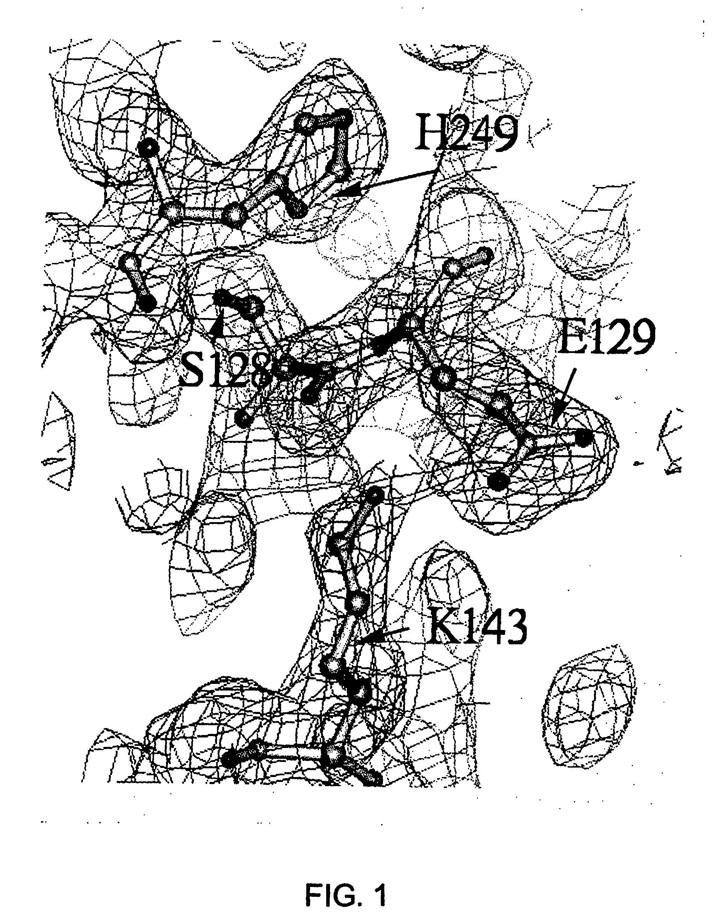

The claimed invention provides crystals of CD40 ligand, as well as the structure of CD40 ligand determined therefrom. Specifically, the claimed invention provides crystals of a fragment of CD40 ligand (116-261) having unit cells which are rhombohedral, and having the following dimensions a=b=77.17 x 10⁻¹⁰ M (Å) and c=90. 46 x 10⁻¹⁰ M (Å), *α= β=* 90 x 10⁻¹⁰ M (Å) and γ = 120 x 10⁻¹⁰M (Å). Almost all of the residues of CD40 ligand fragment, except for residues 116-119 of the N terminus, are well defined in the final electron density map shown in Figure 1. There are areas of weak density for residues 182-186 and 210-220. The current model consists of 142 amino acid residues and 95 water molecules with a crystallographic R factor of 21.8 % and an R_{free} of 29.1% for data between 7.5 x 10⁻¹⁰ M (Å) and 2 x 10⁻¹⁰ M (Å). The Ramachandran diagram shows that 140 out of the 142 amino acid residues have (φ,ψ) angles within the allowed regions The exceptions are residue Cys218, which is involved in the formation of a disulfide bridge, and Lys143.

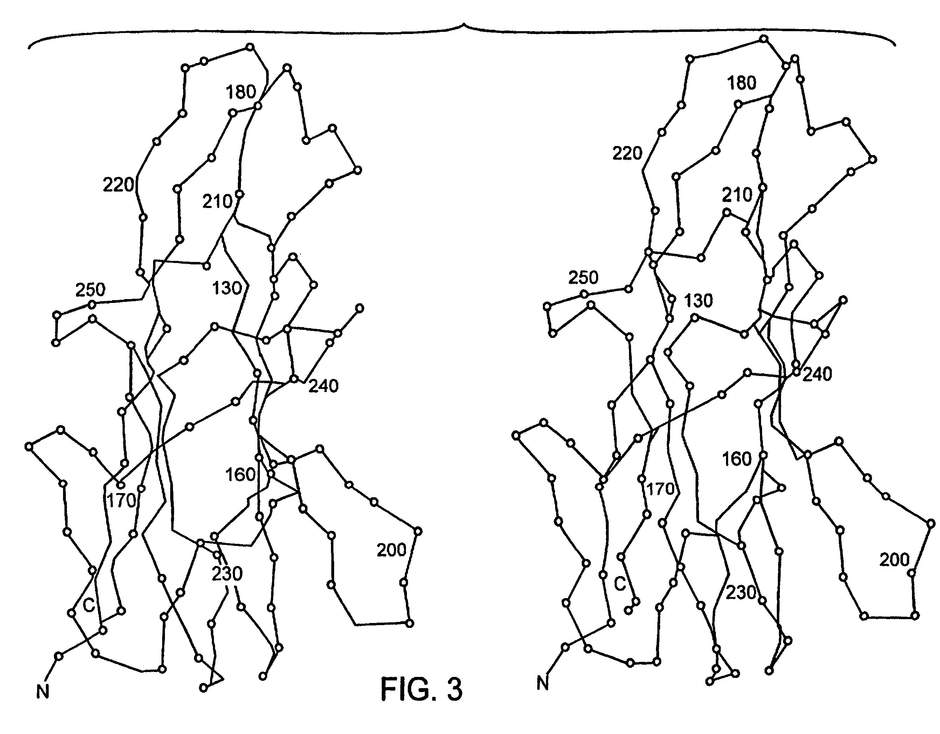

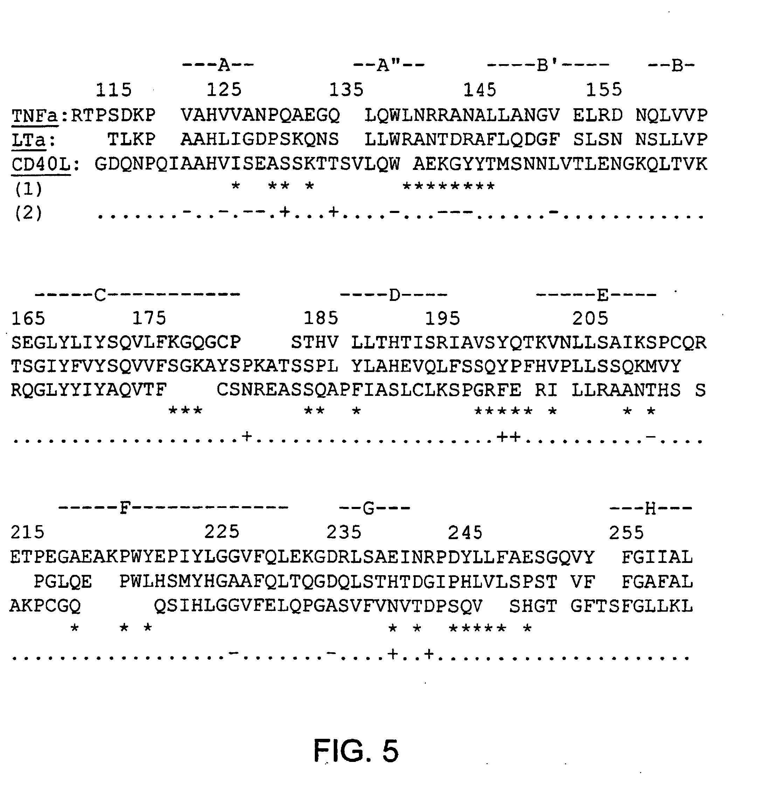

CD40 ligand folds as a sandwich of two β sheets with jellyroll or Greek key topology (Figure 3). The dimensions of the molecule are 25 x 10⁻¹⁰ M (Å) x 30 x 10⁻¹⁰ M (Å) x 50 x 10⁻¹⁰ M (Å). The overall fold is similar to that of TNF-α and LT-α. The notation used herein is that described in Eck et.al., "The Structure of Human Lymphotoxin", J. Biol. Chem., 267, 2119-2112, for the β strands and other structural features. One β sheet consists of strands A"AHCF, and the other of strands B'BGDE. To assess the degree of structural similarity between TNF-α, LT-α and CD40 ligand, the sequences were aligned to maximize the overlap of equivalent β strand residues. The equivalent Cα atoms from the β strands were then used to superimpose the molecular structures. The root mean square (rms) positional deviation of 86 equivalent Cα atoms of superimposed TNF-α and CD40L molecules is 1.10 x 10⁻¹⁰ M (Å). In the case of the LT/CD40L pair, the rms deviation for the 100 equivalent Cα atoms -is 1.03 x 10⁻¹⁰ M (Å). The positions of the residues are highly conserved in the core and β strand regions, but differ significantly in certain loops, such as the AA", CD and EF loops. An alignment of the TNF-α, LT-α and CD40L sequences based on the best structural superimpositions was made. Figure 5. Most residues of the hydrophobic core of CD40L maintain the identity of the character encountered in the equivalent residues of TNF and LT. There are local compensatory, conformational changes to accommodate side-chain changes, such as the substitution of Leu33 of LT-α to Val36 in CD40L, which results in a shift of the side chain of Leu161 to fill the empty space.

Three CD40L molecules form a trimer similar to that observed in the crystal structures of TNF-α and LT-α. The trimer has the shape of a truncated pyramid. The threefold axis of the trimer is approximately parallel to the β strands of each subunit. The interface between the subunits is formed mainly by two tyrosines, two histidines and one leucine. Thus the "aromatic tiling" observed in LT is not as prevalent in CD40L. Tyr170 and His224 from each monomer form an unusual cluster of two triads along the threefold axis of the trimer.

The crystal packing of CD40L trimers was also studied. Each CD40L monomer forms one crystal contact that involves loops DE (residues 198-201), FG (232-233) and BC (64-166) from one molecule and loops CD (182-186), AA" (131-135) and GH (245-246) from the neighboring molecule. Sixteen water molecules are involved in the formation of the contact.

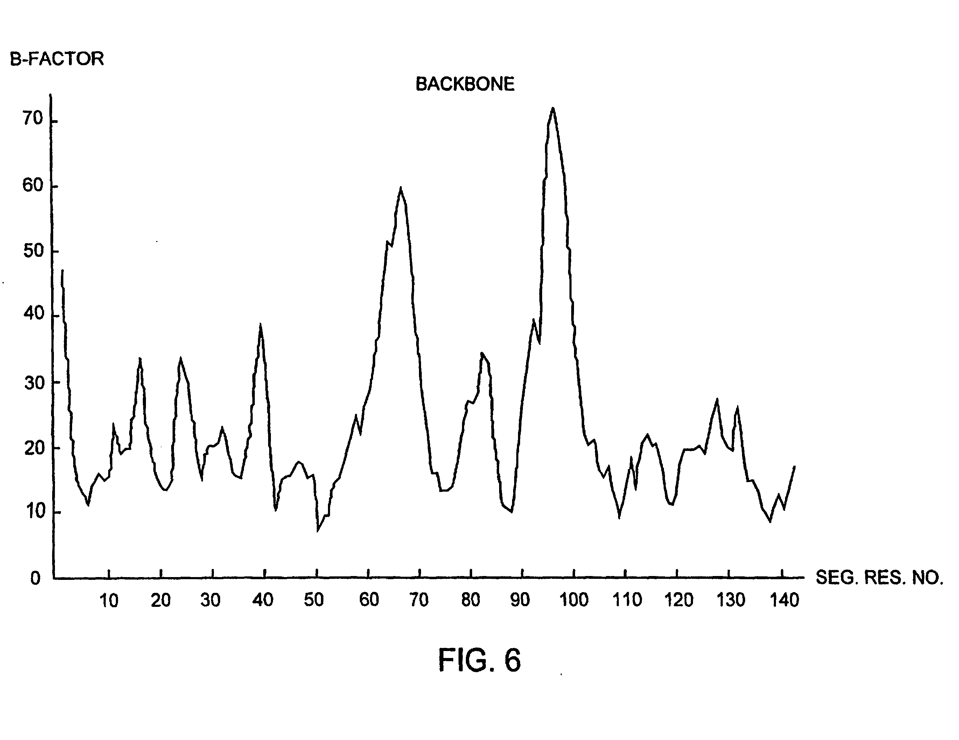

The CD and EF loops of CD40L at the "top" of the molecule are shorter than those of TNF-α and LT-α. However, despite its shorter length, the CD loop contains 1.5 turns of a helix in a similar manner to the equivalent loop in LT. Analogs to LT regions of poor electron density occur within these loops that may indicate disorder. In addition, a plot of temperature factors (Figure 6) suggests that the CD and EF loops are the most mobile parts of the molecule. The C and F strands are linked by a disulfide bridge between Cys178 and Cys218 that may play a role in stabilizing the top of the molecule. A disulfide bridge is also present in TNF but it is located at a different position. Although the AA" loop does not differ much between TNF and LT, in CD40L it has a very different structure. In CD40L the AA" loop is shifted away from the CD40L binding site relative to TNF and LT. The unusual conformation of the AA" loop may be related to its extensive participation in the crystal contact. A short 10 helical structure is found within the GH loop, and β bulge occurs in the middle of strand E. The N and C terminii lie close to each other at the base of the trimer. Residues 116-119 of the N terminus of the construct are poorly defined in the electron-density map, therefore, the first well ordered residue is Pro120. Residues 116-119 presumably constitute part of the stalk connecting the globular domain to the transmembrane segment. This 65-residue stalk is unusually long compared with the equivalent segments in other members of the TNF family.

A single glycosylation site is predicted from analysis of the CD40L sequence. Biochemical experiments revealed that residue Asn240 of the CD40L protein used for the crystallization has an N-linked carbohydrate attached to it. A 2F-Fc map shows electron density in the vicinity of the side chain of Asn240 which presumably corresponds to the attached carbohydrate. However, because the density is not well defined, the current model contains no carbohydrate atoms at this position.

### (b.) Binding Site

By analogy with the LT-TNFR complex crystal structure, the CD40 binding site consists of a shallow groove formed between two monomers. The activation complex of CD40L-CD40 is expected to be similar to the LT-TNFR complex. On the basis of linkage diagrams showing which residue of LT interacts with which residue of TNFR, stretches of the CD40L sequence that were likely to constitute the binding site of CD40L were inferred.

Applicants have determined that the CD40L binding site comprises Arg207 in close physical proximity to at least two hydrophobic residues. More specifically, the binding site comprises at least amino acids Lys143, Arg203, Arg207 and Tyr145. Numerous other residues may be included in the binding site, including, but not limited to, Ile127, Ser128, Glu129, Ala130, Ser131, Thr135, Ser136, Ala141, Glu142, Lys143, Gly144, Tyr145, Tyr146, Cys178, Asn180, Ser185, Gln186, Ala187, Pro188, IIe190, Ala191, Ser192, Ser197, Pro198, Gly199, Arg200, Phe201, Glu202, Arg203, Ile204, Arg207, Ala209, Thr211, Pro217, Cys218, Gly219, Gln220, Glu230, Leu231, Gln232, Asn240, Val241, Thr242, Asp243, Ser245, Val247, Ser24B, His249, Gly250, Thr251, Gly252 and Phe253.

One skilled in the art will appreciate that modifications to the binding site, i.e. substitutions, insertions or deletions, may be made and the binding site will maintain its activity. Thus, it is contemplated that a binding site comprising at least 50 % homology to that defined above is encompassed by the invention. More specifically, it is preferred to have about 60% homology, and most preferred about 75-99% homology. Variations in the percentage of homology to the binding site defined above may alter the binding properties of the resultant entity. In a preferred embodiment, the binding site comprises at least 30 residues from the list defined above.

The binding site was expected to contain residues mostly from the AA" and DE loops. In addition, residues from the CD and GH loops and B strands, C, D, G and H were expected to be involved in CD40 binding. Virtually no sequence conservation exists between CD40L and LT-α or TNF-α in these regions. Although Ser131 of the AA" loop appears to be conserved in the sequence alignment, the structural superimposition shows that it is relatively distant from the equivalent position (Ser38) in LT. The Tyr45 side chain of the AA" loop of CD40L is disordered, but is found in an equivalent position at Arg51 of LT, and, like Arg51 in the LT-α-TNFR complex, may have an extended and well-defined conformation in the complex. On the DE loop, only Phe201 is conserved (equivalent to Phe110 of LT). Phe201 adopts a similar conformation to that of Phe110 in the LT-α-TNFR complex, whereas in the uncoupled LT, Phe110 adopts a different conformation. The Arg200 side chain in the DE loop is involved in a crystal contact. The conformation of loops DE AA" and GH is likely to be affected by the crystal contact, thus, it is expected that their structure will be different in a complex. The conformational changes associated with complex formation in the case of LT, although minor, may be indicative of the magnitude of changes that occur upon CD40 binding to CD40L.

A mixture of both hydrophobic and hydrophilic residues form the surface of the binding site. The LT-TNFR binding interface can be considered as separate upper and lower regions. In a similar manner to LT, the upper region in CD40L has more hydrophobic and non-charged polar residues than the lower region. Arg207 is an important residue, lying in the area where the upper and lower regions connect. The Arg207 side chain adopts an extended conformation pointing towards the putative location of the CD40 receptor, and is surrounded by at least two surface-exposed hydrophobic residues and a serine residue (Ser192). Two of the hydrophobic residues (190 and Phe253) belong to the neighboring subunit, whereas Ile204 is from the same subunit. Murine CD40L has a lysine in the position equivalent to that of Arg207 of human CD40L, and this lysine also seems likely to be surrounded by hydrophobic residues. The hydrophobic environment of the arginine may amplify the strength of a possible electrostatic interaction between this residue and an acidic residue of CD40.

### (c.) Methods of Making a CD40L Crystal

In various embodiments, the claimed invention relates to methods of preparing crystalline forms or CD40L, or fragments thereof by first providing an aqueous solution comprising CD40L or a fragment of CD40L. A reservoir solution comprising a precipitating agent is then mixed with a volume of the CD40L solution and the resultant mixed volume is then crystallized. In certain embodiments, the crystal is derived from an aqueous solution comprising sCD40L(116-261). The concentration of CD40L in the aqueous solution may vary, and is preferably about 1 to about 50 mg/ml, more preferably about 5 mg/ml to about 15 mg/ml, and most preferably, about 10 mg/ml. Similarly, precipitating agents used in the invention may vary, and may be selected from any precipitating agent known in the art. Preferably the precipitating agent is selected from the group consisting of sodium citrate, ammonium sulfate and polyethylene glycol. Any concentration of precipitating agent may be used in the reservoir solution, however it is preferred that the concentration be about 1.0 M to about 1.5 M, more preferably about 1.2 M. The pH of the reservoir solution may also be varied, preferably between about 4 to about 10, most preferably about 7.5. One skilled in the art will understand that each of these parameters can be varied without undue experimentation and acceptable crystals will still be obtained. In practice, once the appropriate precipitating agents, buffers or other experimental variables are determined for any given growth method, any of these methods or any other methods can be used to grow the claimed crystals. One skilled in the art can determine the variables depending upon his particular needs.

Various methods of crystallization can be used in the claimed invention, including, but not limited to, vapor diffusion, batch, liquid bridge, or dialysis. Vapor diffusion crystallization is preferred. See, e.g. McPherson et al., "Preparation and Analysis of Protein Crystals", Glick,. Ed., pp 82-159, John Wiley & Co. (1982); Jancarik et.al., "Sparse matrix sampling: a screening method for crystallization of protein", J. Appl. Cryst. 24, 409-411 (1991), specifically incorporated by reference herein.

In vapor diffusion crystallization, a small volume (i.e. a few milliliters) of protein solution is mixed with a solution containing a precipitating agent. This mixed volume is suspended over a well containing a small amount, i.e. about 1 ml, of precipitating solution. Vapor diffusion from the drop to the well will result in crystal formation in the drop.

The dialysis method of crystallization utilizes a semipermeable size exclusion membrane which retains the protein but allows small molecules (i.e. buffers and precipitating agents) to diffuse in and out. In dialysis, rather than concentrating the protein and the precipitating agent by evaporation, the precipitating agent is allowed to slowly diffuse through the membrane and reduce the solubility of the protein while keeping the protein concentration fixed.

The batch methods generally involve the slow addition of a precipitating agent to an aqueous solution of protein until the solution just becomes turbid, at this point the container can be sealed and left undisturbed for a period of time until crystallization occurs.

Thus, applicants intend that the claimed invention encompass any and all methods of crystallization. One skilled in the art can choose any of such methods and vary the parameters such that the chosen method results in the desired crystals.

### (d.) Use of CD40L Crystal and its Coordinates

The claimed crystals, and coordinates describing them, permit the use of molecular design techniques to design, select and synthesize chemical entities and compounds, including inhibitory compounds or agonists capable of binding to, or associating with, the binding site of CD40L or CD40 in whole or in part.

One approach enabled by this invention is the use of the structural coordinates of CD40L to design chemical entities that bind to or associate with, CD40L and alter the physical properties of the compounds in different ways. Thus, properties such as, for example, solubility, affinity, specificity, potency, on/off rates or other binding characteristics may all be altered and/or optimized.

One may design desired chemical entities by probing a CD40L crystal with a library of different entities to determine optimal sites for interaction between candidate chemical entities and CD40L. For example, high resolution x-ray diffraction data collected from crystals saturated with solvent allows the determination of where each type of solvent molecule sticks. Small molecules that bind tightly to those sites can then be designed and synthesized and tested for the desired activity. Once the desired activity is obtained, the molecules can be further optimized.

The claimed invention also makes it possible to computationally screen small molecule data bases or computationally design chemical entities or compounds that can. bind in whole, or in part, to CD40 or CD40L. They may also be used to solve the crystal structure of mutants, co-complexes, or of the crystalline form of any other molecule homologous to, or capable of associating with, at least a portion of CD40L.

One method that may be employed for this purpose is molecular replacement. An unknown crystal structure, which may be any unknown structure, such as, for example, another crystal form of CD40L, a CD40L mutant, or a co-complex with CD40, or any other unknown crystal of a chemical entity which associates with CD40L which is of interest, may be determined using the structural coordinates of this invention, set forth in Table 1. Co-complexes with CD40L may include, but are not limited to, CD40-CD40L, CD40L-small molecule, and CD40-derived fragments. This method will provide an accurate structural form for the unknown crystal more quickly and efficiently than attempting to determine such information without the claimed invention.

The information obtained can thus be used to optimize potential inhibitors or agonists of CD40L,or CD40, and more importantly, to design and synthesize novel classes of chemical entities which will affect the relationship between CD40L and CD40.

The design of compounds that inhibit or agonize CD40L according to this invention generally involves consideration of at least two factors. First, the compound must be capable of physically or structurally associating with CD40L or CD40. The association may be any physical, structural, or chemical association, such as, for example, covalent or noncovalent bonding, van der Waals interactions, hydrophobic or electrostatic interactions.

Second, the compound must be able to assume a conformation that allows it to associate with CD40 or CD40L. Although not all portions of the compound will necessarily participate in the association with CD40 or CD40L, those non-participating portions may still influence the overall conformation of the molecule. This in turn may have a significant impact on the desirability of the compound. Such conformational requirements include the overall three-dimensional structure and orientation of the chemical entity or compound in relation to all or a portion of the binding site.

The potential inhibitory or binding effect of a chemical compound on CD40 or CD40L may be analyzed prior to its actual synthesis and testing by the use of computer modeling techniques. If the theoretical structure of the given compound suggests insufficient interaction and association between it and CD40 or CD40L, the need for synthesis and testing of the compound is obviated. However, if computer modeling indicates a strong interaction, the molecule may then be synthesized and tested for its ability to bind to CD40 or CD40L. Thus, expensive and time consuming synthesis of inoperative compounds may be avoided.

An inhibitory or other binding compound of CD40 or CD40L may be computationally evaluated and designed by means of a series of steps in which chemical entities or fragments are screened and selected for their ability to associate with the individual binding sites of CD40L.

Thus, one skilled in the art may use one of several methods to screen chemical entities or fragments for their ability to associate with CD40L and more particularly, with the individual binding sites of sCD40L(116-261). This process may begin by visual inspection of, for example, the binding site on a computer screen based on the CD40L coordinates in Table 1. Selected fragments or chemical entities may then be positioned in a variety of orientations, or "docked", within an individual binding pocket of CD40L. Docking may be accomplished using software such as Quanta and Sybyl, followed by energy minimization and molecular dynamics with standard molecular mechanics force fields, such as CHARMM and AMBER.

Specialized computer programs may be of use for selecting interesting fragments or chemical entities. (GRID, available from Oxford University, Oxford, UK; MCSS or CATALYST, available from Molecular Simulations, Burlington, MA; AUTODOCK, available from Scripps Research Institute, La Jolla, CA; DOCK available from University of California, San Francisco, CA., XSITE, University College of London, UK.)

Once suitable chemical entities or fragments have been selected, they can be assembled into an inhibitor or agonist. Assembly may be by visual inspection of the relationship of the fragments to each other on the three-dimensional image displayed on a computer screen, in relation to the structural coordinates disclosed herein.

Alternatively, one may design the desired chemical entities "de novo", experimentally, using either an empty binding site, or optionally including a portion of a molecule with desired activity. Thus, for example, one may use solid phase screening techniques where either CD40L or a fragment thereof, or candidate chemical entities to be evaluated are attached to a solid phase thereby identifying potential binders for further study or optimization.

Basically, any molecular modeling techniques may be employed in accordance with the invention; these techniques are known, or readily available to those skilled in the art. It will be understood that the methods and compositions disclosed herein can be used to identify, design or characterize not only entities which will associate or bind to CD40L, but alternatively to identify, design or characterize entities which, like CD40L, will bind to CD40 thereby disrupting the CD40L-CD40 interaction. The claimed invention is intended to encompass these methods and compositions broadly.

Once a compound has been designed or selected by the above methods, the efficiency with which that compound may bind to CD40L may be tested and optimized using computational or experimental evaluation. Various parameters can be optimized depending on the desired result. These include, but are not limited to, specificity, affinity, on/off rates, hydrophobicity, solubility and other characteristics readily identifiable by the skilled artisan.

Thus, one may optionally make substitutions, deletions, or insertions in some of the components of the chemical entities in order to improve or modify the binding properties. Generally, initial substitutions are conservative, i.e the replacement group will have approximately the same size, shape, hydrophobicity and charge as the original component.

The present invention also enables the design of mutants of CD40L and the solving of their crystal structure. More particularly, the claimed invention enables one skilled in the art to determine the location of binding sites and interfaces, thereby identifying desirable sites for mutation.

For example, mutation may be directed to a particular site or combination of sites on the CD40L, by replacing or substituting one or more amino acid residues. Such mutants may have altered binding properties which may or may not be desirable.

The mutants may be prepared by any methods known in the art, such as for example, site directed mutagenesis, deletion or addition, and then tested for any properties of interest. For example, mutants may be screened for an altered charge at a particular pH, tighter binding, better specificity etc.

The CD40L structure suggests that most, if not all, of the naturally occurring single-site HIGMS mutations affect the folding and stability of the protein rather than the binding site directly. Indeed, more severe HIGMS mutations involving deletions or other drastic changes would be expected to perturb the structure much more than single-site mutations. In addition, only two of the six binding-site residues selected for mutagenesis were found to be critical for CD40 binding.

A similar low frequency of hits was found in an analogous mutational study of LTα. Van Ostade et al., "Structure activity studies of human tumour necrosis factors.", Protein Engineering 7: 5-22 (1994). This observation raises the question of whether mutations of individual CD40L binding-site residues are generally sufficient to completely disrupt CD40L-CD40 binding. If insufficient, CD40-mediated cellular signaling will continue and the mutation will be clinically undetectable. This behavior is consistent with the fact that, in the LTα-receptor complex structure, as many as 38 LTα residues are involved in the binding interface. The interface is quite extensive (520 10⁻¹⁰ M² (Å²)) and is presumably of similar dimensions in the CD40L-CD40 complex. It is expected, therefore, that each residue only contributes a small fraction of the binding energy. However, the interaction of the human growth hormone with its receptor has also been shown to involve a large surface area, yet relatively few key residues contribute most of the binding energy.

Additionally, the claimed invention is useful for the optimization of potential small molecule drug candidates. Thus, the claimed crystal structures can be also be used to obtain information about the crystal structures of complexes of the CD40 ligand and small molecule inhibitors. For example, if the small molecule inhibitor is co-crystallized with CD40 ligand, then the crystal structure of the. complex can be solved by molecular replacement using the known coordinates of CD40 ligand for the calculation of phases. Such information is useful, for example, for determining the nature of the interaction between the CD40 ligand and the small molecule inhibitor, and thus, may suggest modifications which would improve binding characteristics such as affinity, specificity and kinetics.

The detrimental effects of several HIGMS mutations on CD40L appear to be caused by destabilizing its structure. For example, Trp140 is a large hydrophobic residue buried inside the protein and removal of its side chain in the HIGMS mutations Trp140→Gly and Trp140→Arg will obviously lead to destabilization of the structure. The valine affected by the Val126→Ala mutation also participates in forming the hydrophobic core. Leu155 is not completely buried but lies in the middle of β strand B. Introduction of proline at this position, as in the Leu155→Pro HIGMS mutation, may disrupt the formation of the strand. The behavior of the HIGMS mutation Ala235→Pro can be similarly explained. Substitution of Gly144 in the HIGMS mutation Gly144→Glu may result in loss of the conformational freedom necessary at that position to form the corner of the AA" loop. Consequently, the positioning of the adjacent residues, Lys143 and Tyr145, which are known to be involved in binding, is disrupted. An interesting observation is that all of the above mentioned residues appear to be clusters: Trp140, Leu155 and Val126 participate in the formation of an area of the hydrophobic core partially formed by strands A, A", B' and B. Lys143, Gly144 and Tyr145 are exposed surface residues of the AA" loop lying very close to the above mentioned hydrophobic residues. This suggests that the CD40-binding capacity is very sensitive to perturbations in this area of CD40L.

HIGMS mutations Ala123→Glu, Gly227→Val and Thr211→Asp affect residues that are involved in formation of the subunit interface. Ala123 lies near the bottom of the trimer and is within van der Waals distance of three hydrophobic residues (Leu168, Val228 and Leu261) of the neighboring subunit. Gly227 packs against Tyr172 of the neighboring subunit and Thr211 lies on the EF loop close to the intersubunit interface where it appears to interact with Arg181 from the neighboring subunit. Mutations that introduce larger side chains at these positions (e.g. Thr211→Asp) would be expected to disturb the intersubunit packing and possibly prevent trimeritation. Thus, it appears that most of the single-site HIGMS mutations affect the folding and stability of CD40L rather than playing a direct role in CD40 binding. In addition, mutations of only two of the nine targeted surface residues appear to affect CD40 binding.

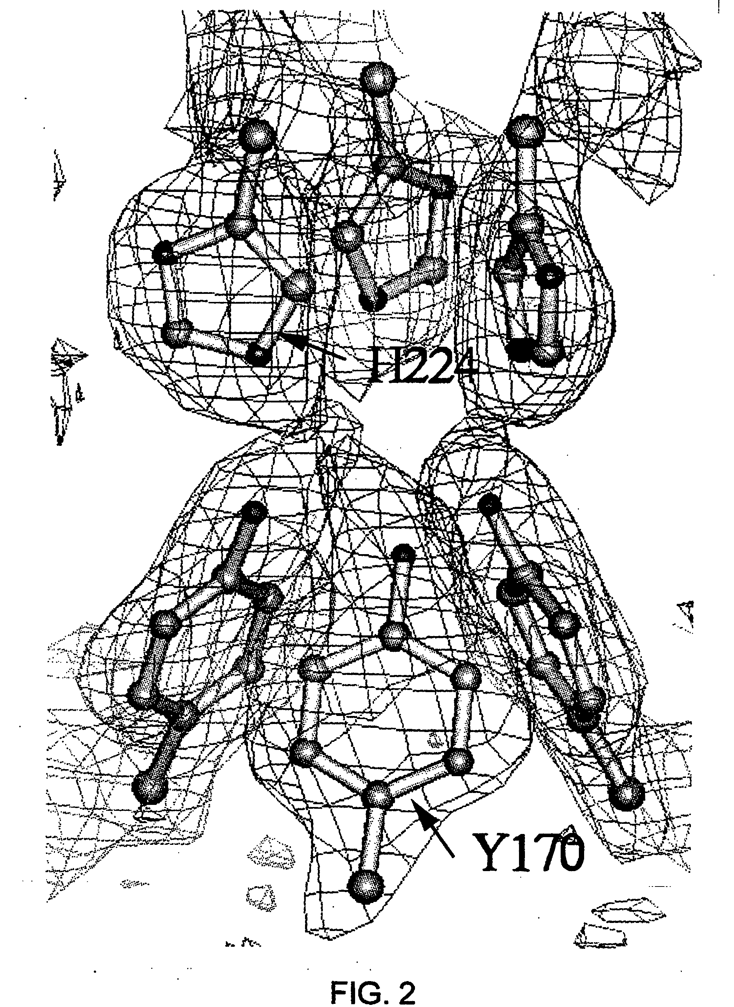

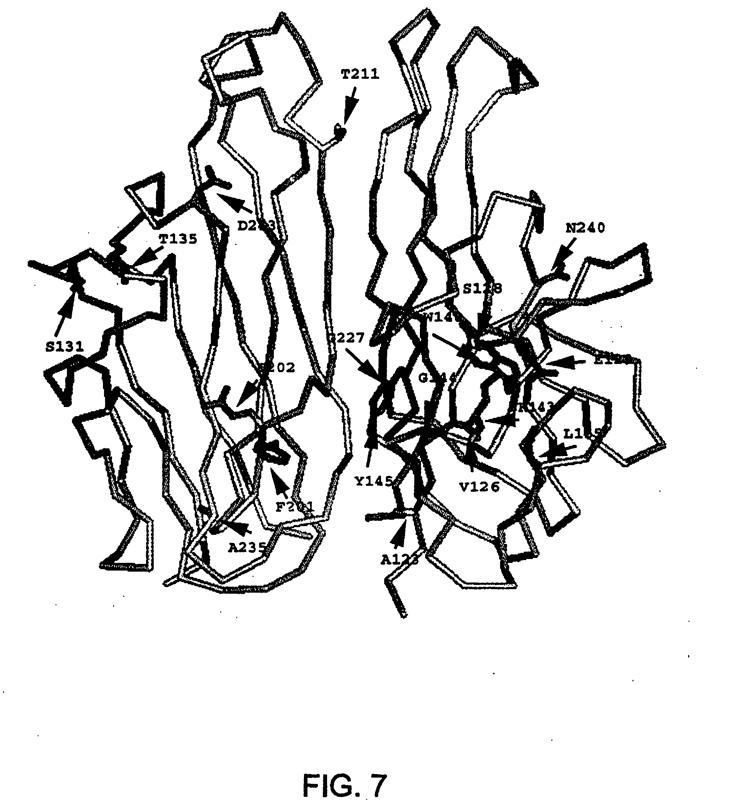

Mutagenesis studies on CD40L have shown that residues Tyr145 and Lsy143 are directly involved in CD40 binding. The crystal structure shows that both residues lie on the AA" loop and are surface-exposed. Figure 7. The equivalent loop in the LT-TNFR crystal structure is involved in several ligand receptor contacts suggesting similarities in binding. Tyr145 lies at the corner of the loop and its side chain has no visible electron density which suggests that it is disordered. Atom N1 of Lys143 forms a hydrogen bond with 0δ1 of Glu129. Figure 2. The electron density map also shows that the side chain of Lys143 has an additional conformation. Ser128 and Glu129, two residues involved in the HIGMS. mutation Ser128→Arg, Glu129→Gly lie close to Lys143 and are well defined in the map. Ser128 is almost completely buried, and its side-chain hydruxyl group is within hydrogen bonding distance of His249. It has been shown that the Glu129-Gly substitute found in the HIGMS mutation affects a solvent accessible residue that might participate in binding to CD40. Bajorath et al., "Identification of Residues on CD40 and its Ligand which are Critical for the Receptor-ligand interaction", Biochemistry 34, 1833-1844 (1995), specifically incorporated herein by reference. One possible candidate for it is the exposed residue Lys143, and loss of its hydrogen bond to Glu129 may destabilize its conformation resulting in reduced CD4D binding. All of these residues are conserved in the murine CD40L sequence.

Five additional single-site mutations, in which residues Ser131, Asn180, Phe201 Glu202 and Asn240 (located at the binding site) were substituted by alanine, established that these residues are not critical for CD40 binding. In addition, two other mutations at residue positions outside of the binding site (Thr135 and Asp243) were also shown not to affect binding. According to the structure, Ser131 and Thr135 lie on the AA" loop and their side chains are not exposed. Asn240, the site of glycosylation, is exposed to the solvent. Because the attached sugar would be expected to make Asn240 unavailable for interaction with CD40, we conclude that it is probably not involved in CD40 binding, in agreement with the mutagenesis results. Asp243 is a semi-exposed residue and it makes a hydrogen bond to Asn186. Asn180 is an exposed residue at the top of the molecule. Its side chain makes hydrogen bonds to residues 183 and 216. Phe201 and Glu202 both lie on the DE loop in the binding site area and are exposed to the solvent.

### C. EXAMPLES

### 1. Determination of Crystal Structure of SCD40L(116-261)

### A. Crystallization

Buffer chemicals were purchased from Fisher (Boston, MA). Crystallization condition screenings were done with the Crystal Screen^{TM} kit from Hampton Research (Riverside, CA). A soluble fragment of the extracellular domain of human CD40 ligand containing amino acid residues Gly116 to the C-terminal residue Leu261 was produced in soluble form and purified as follows:

The gene encoding the SCD40L (116-261) sequence of amino acids G116-L261 was cloned into the *Pichia pastoris* expression vector pWS106 and the SCD40L(116-261) protein was expressed by using standard protocols. Peitsch et al., "A B-D Model for the CD40 Ligand Predicts That it is a Compact Trimer Similar to the Tumor Necrosis Factors.", Int. Immunol. 5, 233-238; (1993). pWS106 is a variant of vector pPIC9 (Invitrogen) with the NcoI site at 3634 nt in the 3'-AOX1 flanking region deleted by site directed mutagenesis. The cells were lysed and the medium was dialyzed overnight with 20mM Tris-HCl, pH 6.8 and loaded onto an SP (Pharmacia) column. The bound sCD40L was eluted by 2xPBS, pH 7.2 and buffer-exchanged to 1xPBS.

The protein stock was made available as a solution of 8 mg/ml SCD40L in PBS (20.44 g/ℓ Na₂HPO₄, 7.73 g/ℓ Na₂HPO₄-H₂O, 87.66 g/lt NaCI) buffer. Crystals were grown by the vapor diffusion method. (see Jancarik et.al.). In order to find conditions of crystallization, an incomplete factorial screen was set up. In a typical experiment; protein solution was mixed with an equal volume of reservoir solution and a drop of the mixture was suspended under a glass cover slip over the reservoir solution. Crystals were grown out of 1.4 M Na Citrate, 50 mM Na Hepes pH 7.5 reservoir solution. The crystals are shaped as cubes, are easy to reproduce and can reach maximum dimensions of almost 1mm on each side (optimal concentration of precipitant to yield the biggest crystals is 1.2 M Na Citrate). Variation of pH between 7 and 8 did not affect crystal quality. Macroseeding techniques were also successful, although not necessary, to get crystals of sufficient quality for data collection. Crystal composition was assayed after washing, dissolution in water and SDS electrophoresis. The gel showed a strong band of about 20,000 daltons and a much fainter band of 17,000 daltons. The fainter band corresponds to non-glycosylated protein while the stronger band corresponds to the glycosylated form.

Those of skill in the art will appreciate that the aforesaid crystallization conditions can be varied. By varying the crystallization conditions, other crystal forms of sCD40L may be obtained. Such variations may be used alone or in combination, and include: varying final protein concentrations between 5 mg/ml and 35 mg/ml; varying the SCD40L to precipitant ratio; varying citrate concentrations between 1.2 M and 2.0 nM; varying pH ranges between 5.5 and 9.5; varying HEPES concentrations between 5 and 395 mM; varying the concentration or type of detergent; varying the temperature between -5 °C and 30 °C; and crystallizing SCD40L by batch, liquid bridge, or dialysis method using the above conditions or variations thereof. See McPherson, A. (1982). Preparation and Analysis of Protein Crystals. (Click, ed.) pp. 82-159, John Wiley & Co., N.Y., specifically incorporated by reference herein.

### B. Data collection and processing

Crystals were mounted inside capillary tubes and tested for diffraction capacity on the X-ray beam of an Elliot GX-13 generator. Oscillation photographs showed strong diffraction to high resolution. Initial inspection of the photographs suggested an almost cubic lattice.

A large crystal (0.8 x 0.8 x 0.8 mm) was equilibrated gradually in a cryoprotectant solution of 20% glycerol., 1.2 M Na Citrate , 50 mM Na Hepes pH 7.5, mounted on a loop and immediately frozen in a -150 °C liquid nitrogen gas stream. The technique of freezing the crystals essentially immortalizes them and produced a much higher quality data set. A native X-ray data set up to 1.75 x 10⁻¹⁰ M (Å) resolution was collected by using a Nicolet/Siemens multiwire area detector (Siemens, Inc.). The data were integrated and reduced using BUDDHA (25) and the CCP4 program package (The SERC (UK) Collaborative Computing Project No 4; Daresbury Laboratory, UK 1979). The data collection required about 5 days.

Data processing suggested a rhombohedral unit cell with approximate cell dimensions a=b=c=55 x 10⁻¹⁰ M (Å) and α=β=γ=91. To assist calculations, a hexagonal unit cell is defined instead with dimensions a=b=77.17 x 10⁻¹⁰ M (Å), c=90.46 x 10⁻¹⁰ M (Å), γ=120 degrees. Merging of data suggested that the space group is R3. If space group R3 is assumed, then R_{merge} is 6.7%. Assuming space group R32, R_{merge} is 16.6%. Table 1 contains information for the data set obtained.

Calculation of the Matthews volume gives VM= 533,610 A³/Z*MW=2.97 assuming Z=9 (#of asymmetric units in unit cell) and MW=20,000 daltons. Eck et.al., J. Biol.Chem. 267, 2119-2112 (1992), specifically incorporated herein by reference. Thus, it was not clear initially whether there were one or two monomers in the asymmetric unit.

### C. Molecular replacement

All subsequent molecular replacement and refinement computing was done with the XPLOR program package. Molecular graphics manipulations were done with QUANTA (Molecular Simulations, Inc.) software. Both XPLOR and QUANTA were run on a Silicon Graphics Indigo2 computer workstation.

To investigate whether non-crystallographic symmetry was present, the self rotation function was calculated with 8-4 x 10⁻¹⁰ M (Å) data. A very strong peak was found for φ=90°, ψ=90°, κ=180°, which corresponds to the crystallographic 3-fold axis. This coincides with the 3-fold axis of the trimer of SCD40L. Also, less strong peaks appeared for φ=0°, ψ=30°, κ=180° and φ=0° ψ=90°, κ=180° and φ=0°, ψ=150°, κ=180° corresponding to 2-fold axes perpendicular to the 3-fold. None of these axes corresponded to non-crystallographic symmetry.

A 3-dimensional model of the human SCD40L was constructed by using the murine CD40L model as a framework, using QUANTA protein homology modeling software. The model was used as a probe for molecular replacement calculations.

Calculation of the cross rotation function by using a 2.5° angle grid and 8-4 x 10⁻¹⁰ M (Å) data produced a strong peak 4.5σ above the mean, at Euler angles θ₁=234.5°, θ₂=5.0°, θ₃=234.5°. Rotation of the model according to that solution and subsequent generation of symmetry-related molecules corresponding to the 3-fold crystallographic symmetry produces a trimer with the 3-fold axis parallel to the z axis, as expected. The exact rotation solution was found with Patterson correlation refinement. No significant second peak was observed in the rotation function.

In order to conduct the translation search, the model probe was rotated according to the first peak of the rotation search and translated so that the center of mass of the trimer would lie on the z axis. This was assumed to be close to the expected position since it appeared that the 3-fold axis of the trimer lay on the z axis of the unit cell. Initial attempts to find a clear peak in the translation function on the xy plane failed. Trimmed model probes, which contained mostly core residues whose structure was more likely to be conserved among different members of the TNF family, produced translation functions with a clustering of peaks at locations close to the expected ones. One of those trimmed models containing all backbone atoms as well as side chain atoms for residues 8-12, 53-62, 90-93, 110-114 and 141-146 produced a peak with a correlation coefficient of 3.30 above the mean at x=0.053, y=0.053. This peak was the highest on the list of peaks and was close to the expected position.

By generating symmetry related molecules and displaying them with computer graphics it was found that they packed satisfactorily in the unit cell and that there was not enough space for a second molecule. Thus, we concluded that there was only one molecule in the asymmetric unit. The existence of 2-fold symmetry in the self rotation function apparently is a consequence of internal symmetry within the molecule, possibly related to the high content of parallel strands. In fact, the self rotation function calculated from structure factors derived from the model shows peaks corresponding to 2-fold symmetry.

### D. Model building and crystallographic refinement

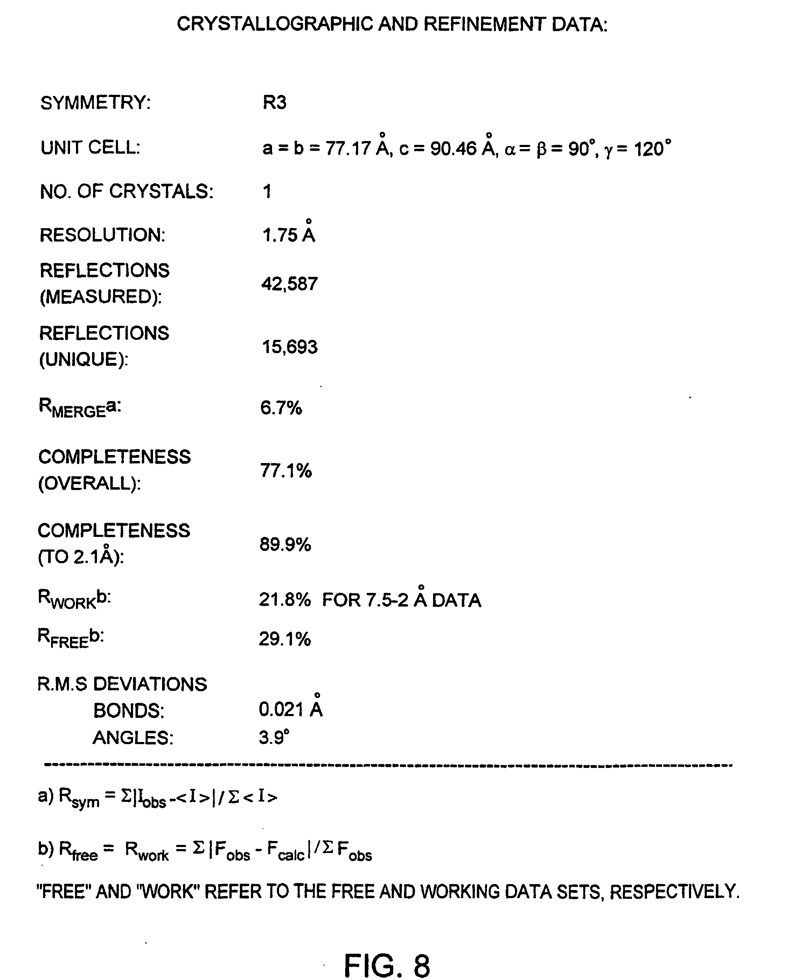

The param19.pro stereochemical parameter set of XPLOR package was used for all refinement calculations. The partial model that was used to find the solution of the translation function was subjected to rigid body, refinement by using 7.5-2.5 x 10⁻¹⁰ M (Å) data. The R and R_{free} (29) factors after the initial rigid body refinement were 49.9% and 51,4% respectively. The test data set used for the calculation of R_{free} contained 10% of the data. The partial model was subjected to 40 steps of conventional positional refinement and a cycle of simulated annealing with an initial temperature of T=2500 K. The R and R_{free} factors dropped to 31.2% and 43.1% respectively. To reduce model bias, a partial model was used for map calculation and refinement. The resolution range used was 7.5-2.5 x 10⁻¹⁰ M (Å). Simulated annealing omit maps (30) calculated by consequently omitting 10% of the model each time showed which parts of the model could be used for phasing. Thus, the model was modified to include only residues sufficiently well defined in annealed omit maps. The initial model included 815 atoms out of a total of 1374 atoms of the complete model. 3Fₒ -2F_{c} maps were used for cycles of model building and refinement. Typically, cycles consisted of model building, positional refinement and B-factor refinement. Occasionally simulated annealing was performed. As the phases improved, more atoms were added into the model. Initially, grouped B-factors were assigned for each strand (one for main chain and the one for side chain atoms). Later, grouped B-factors and finally individual atomic B-factors where refined for each residue. Only manual structure modifications that resulted in lower R_{free} after refinement were accepted. When R and R_{free} reached 31.1% and 36.4% respectively, the resolution was extended to 2.25 x 10⁻¹⁰ M (Å) and finally to 2 x 10⁻¹⁰ M (Å). Water molecules were added by using the X-solvate utility of QUANTA 4.1. Both occupancies and temperature factors were refined for the water molecules. Figure 8 summarizes information regarding crystallographic data and refinement. Table 1 lists the atomic coordinates of sCD40L (116-261).

The coordinates of the crystal structure of a sCD40L may be used in the structure-based design of small molecule inhibitors of CD40L, computational drug design and iterative structure optimization.

### a. Computational drug design

Small molecule inhibitors can be designed using computational approaches. These approaches are also known as de novo drug design. In brief, the crystal structure coordinates of the SCD40L are the input for a computer program, such as DOCK. Programs such as DOCK output a list of small molecule structures that are expected to bind to CD40L. These molecules can then be screened by biochemical assays for CD40L binding.

Typically, biochemical assays that screen molecules for their ability to bind to CD40L are competition-type assays. In such assays, the molecule is added to the assay solution and the degree of inhibition is measured using conventional methodology.

### b. Iterative cycles of structure optimization

The crystal structures of complexes formed between SCD40L and small molecule inhibitors may be solved. In brief, 1 small molecule inhibitors are typically found using the crystal structure coordinates of a sCD40L either by the computational approaches mentioned above or by the screening of small molecule libraries. The small molecule inhibitor is then co-crystallized with sCD40L and the crystal structure of the complex is solved by molecular replacement. Molecular replacement requires the coordinates of a sCD40L for the calculation of phases. The information collected from these experiments can be used to optimize the structure of small molecule inhibitors by clarifying how small molecules interact with the protein target. This suggests ways of modifying the small molecule to improve its physicochemical properties, such as affinity, specificity, and kinetics with regard to the CD40L target.

In addition to being necessary for computational drug design and structure optimization, the crystal coordinates of sCD40L are useful for analyzing the CD40L binding site. Through such analysis, it was determined that a particularly attractive region for drug targeting is in the vicinity of Arg207. Arg207 is situated in a region surrounded by three hydrophobic residues and one moderately hydrophobic residue. This grouping appears to demark one wall of a binding groove formed between two CD40L monomers. While not wishing to be bound by theory, it appears that Asp84, or Glu117 of CD40 probably interacts with CD40L at this site. The hydrophobic residues that surround Arg207 may result in increased strength of electrostatic interactions between Arg207, Asp84 and Glu117 of CD40. Specifically, it appears that Glu117 and Asp84 are important. The above observations and hypotheses suggest that this region may contribute significantly to the binding energy of CD40L/CD40 interactions, and therefore, is an attractive target for inhibitor design. Site mutations studies can be used in conjunction with the above-described processes to further define the binding site.

It will be apparent to those skilled in the art that various modifications and variations can be made in the methods and compositions of the present invention without departing from the spirit or scope of the invention. Thus, it is intended that the present invention cover the modifications and variations of this invention provided that they come within the scope of the appended claims and their equivalents.

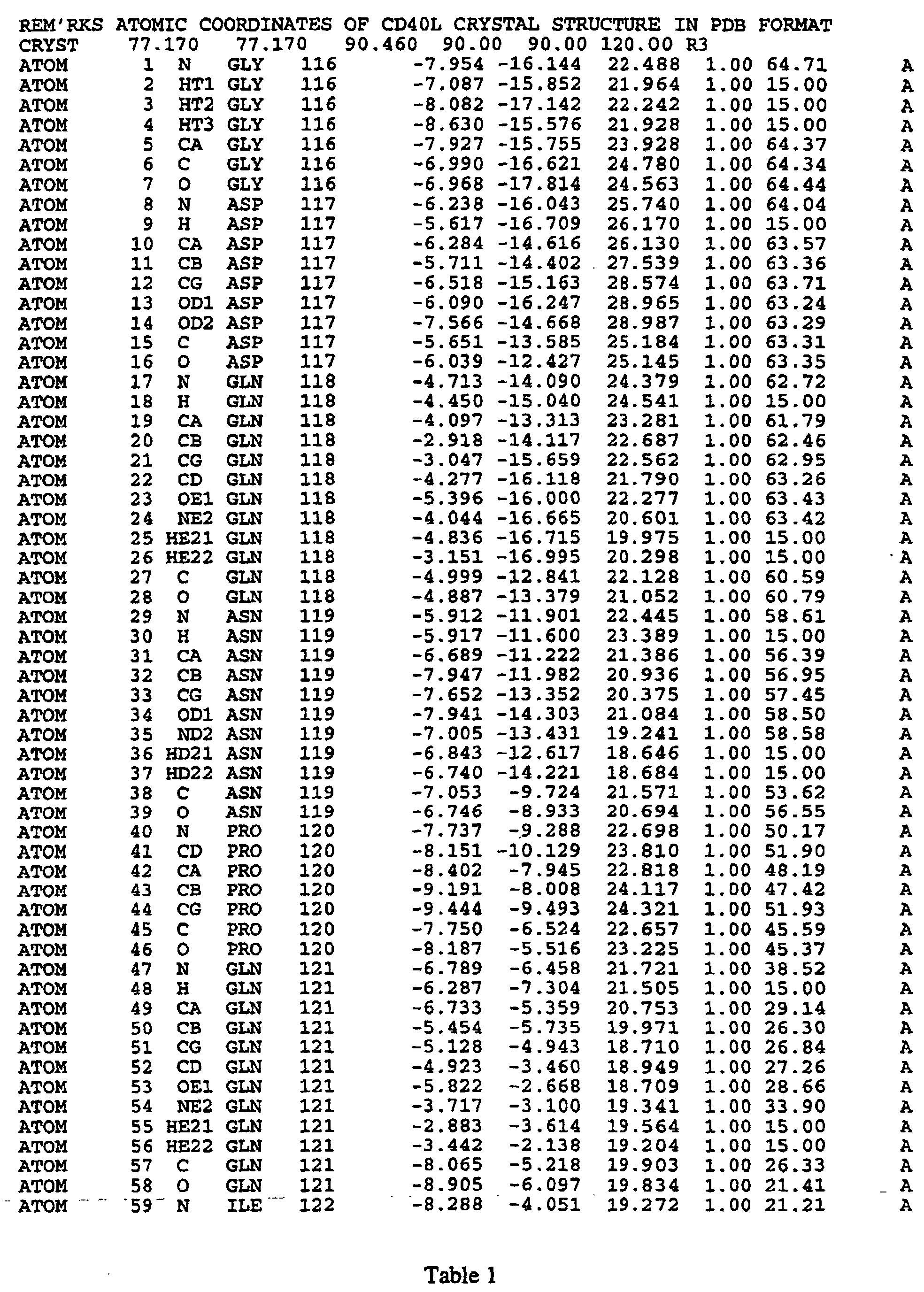

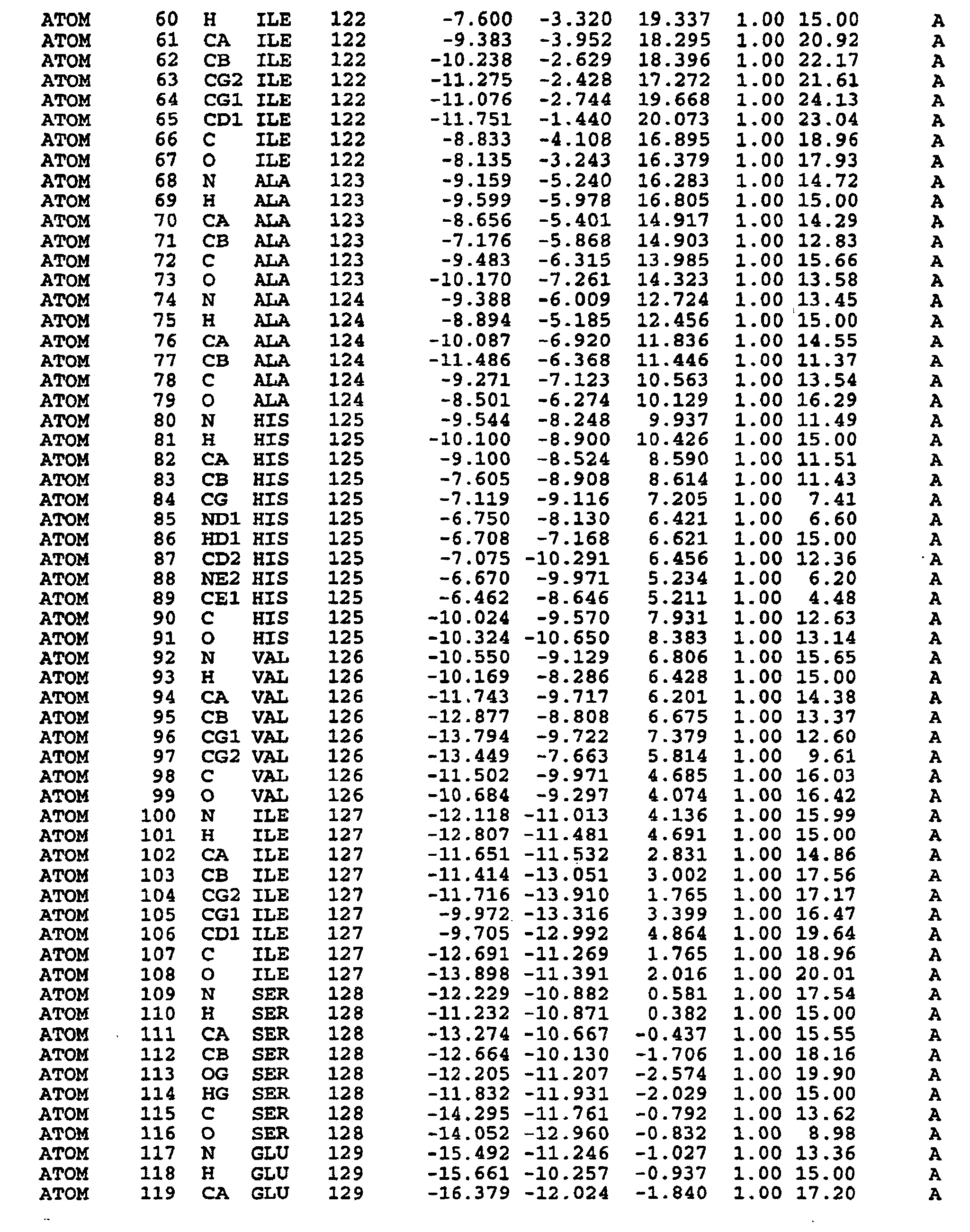

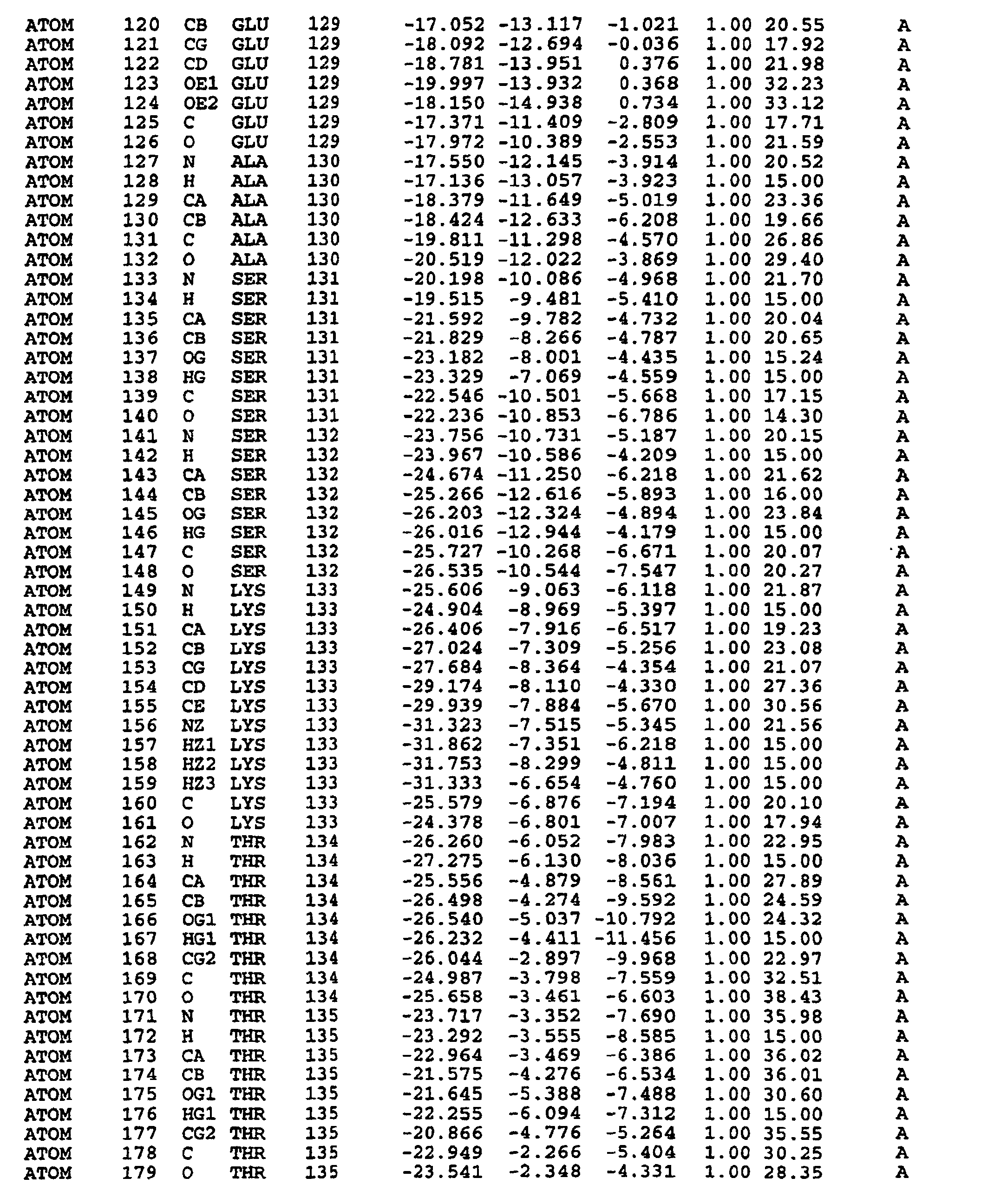

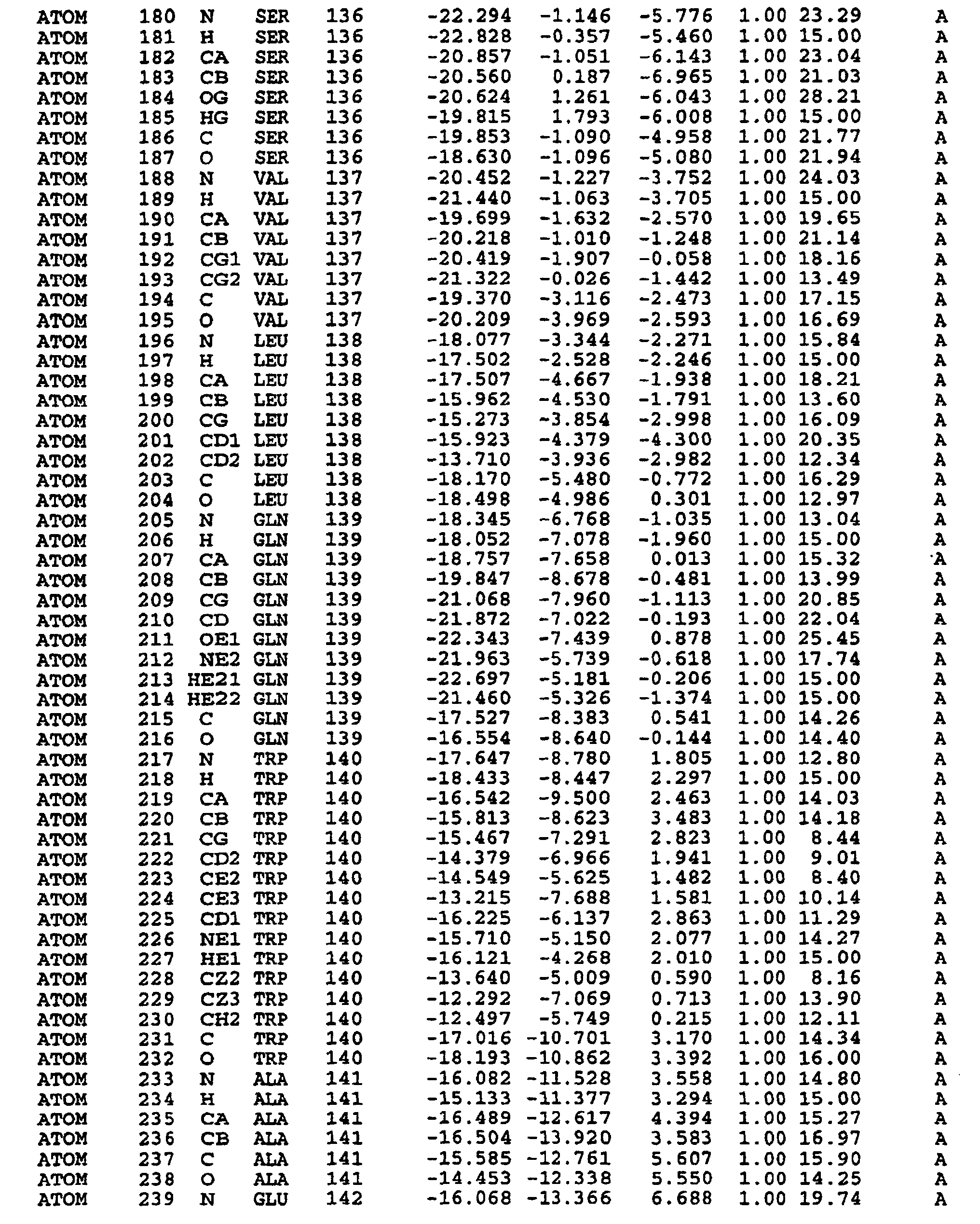

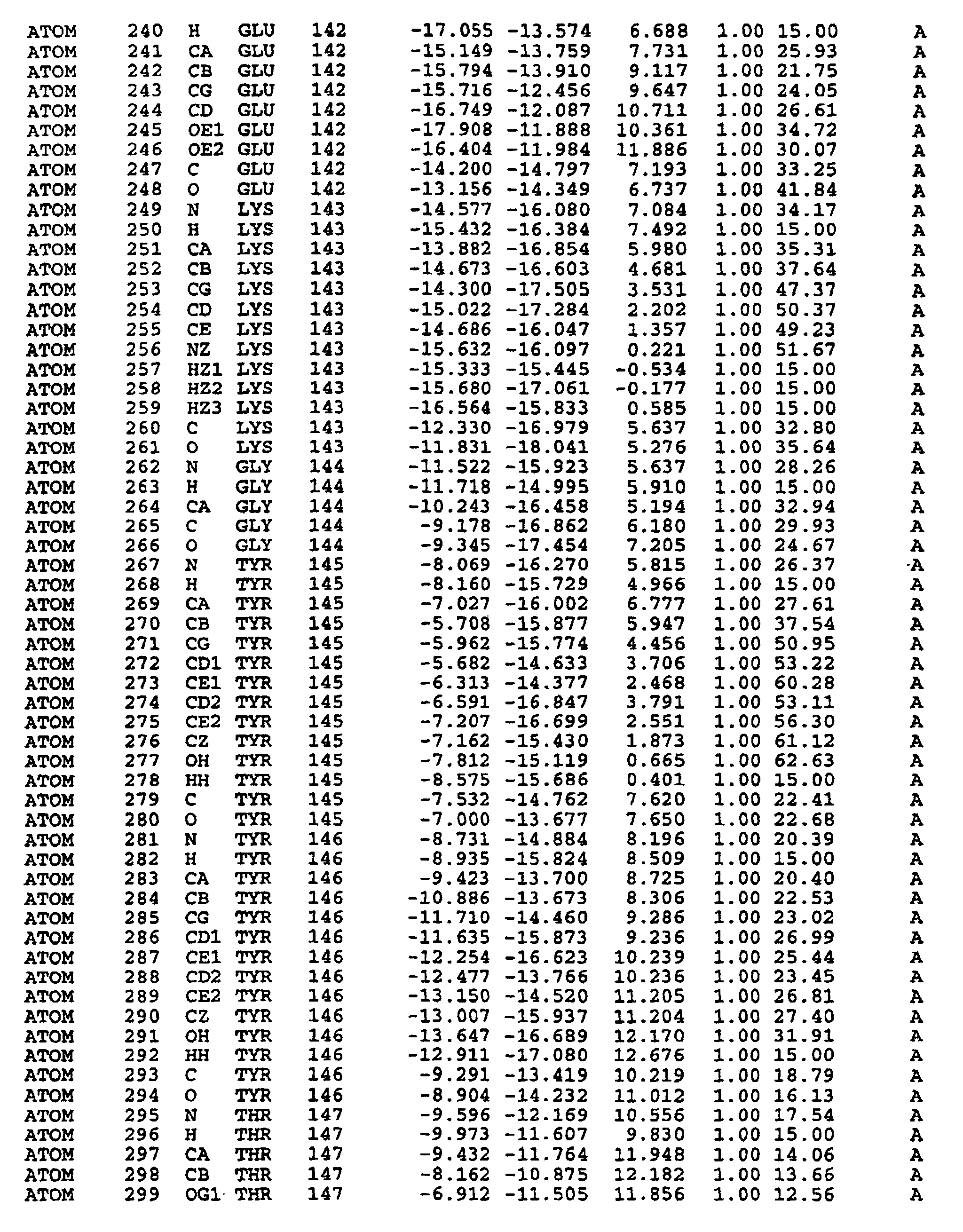

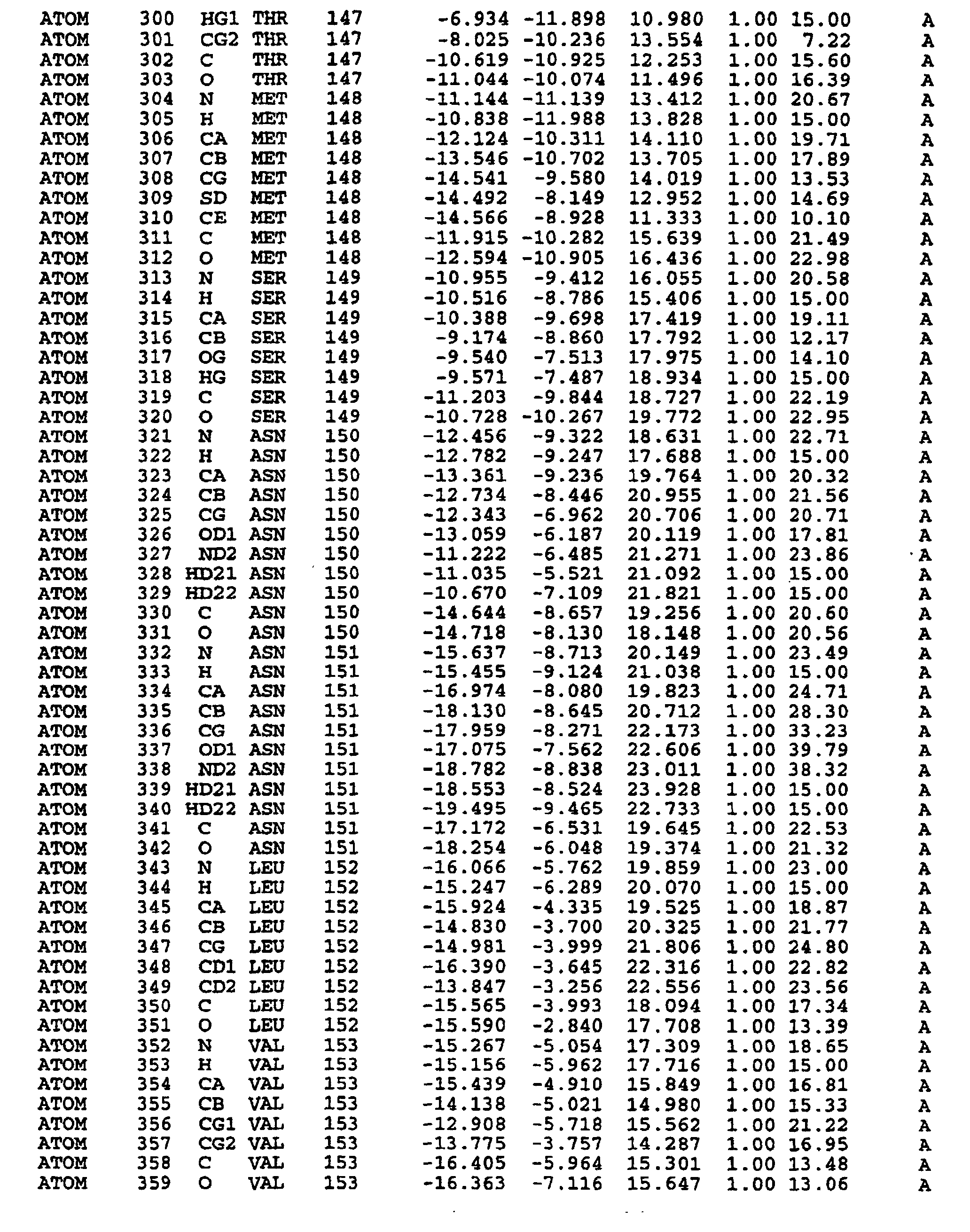

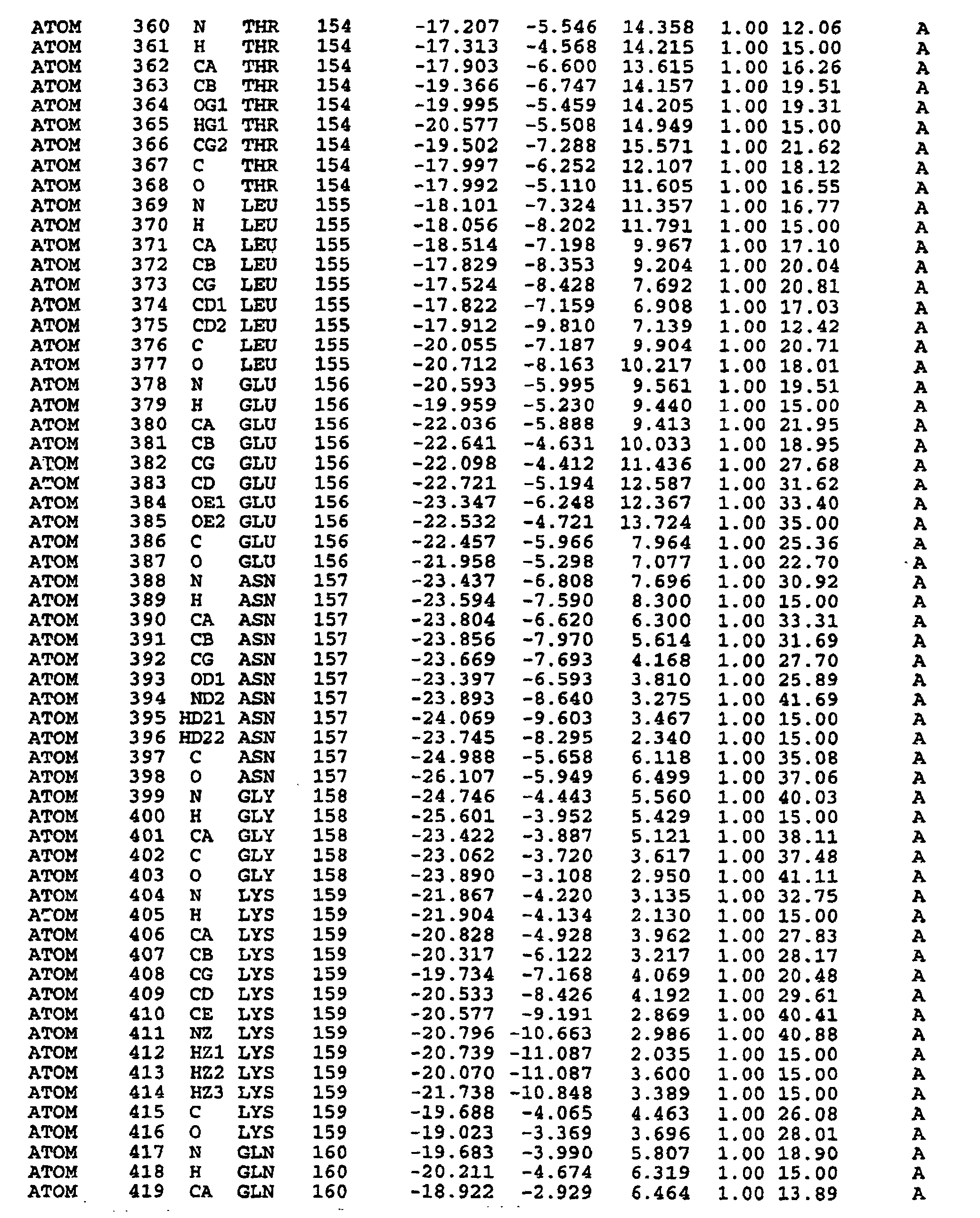

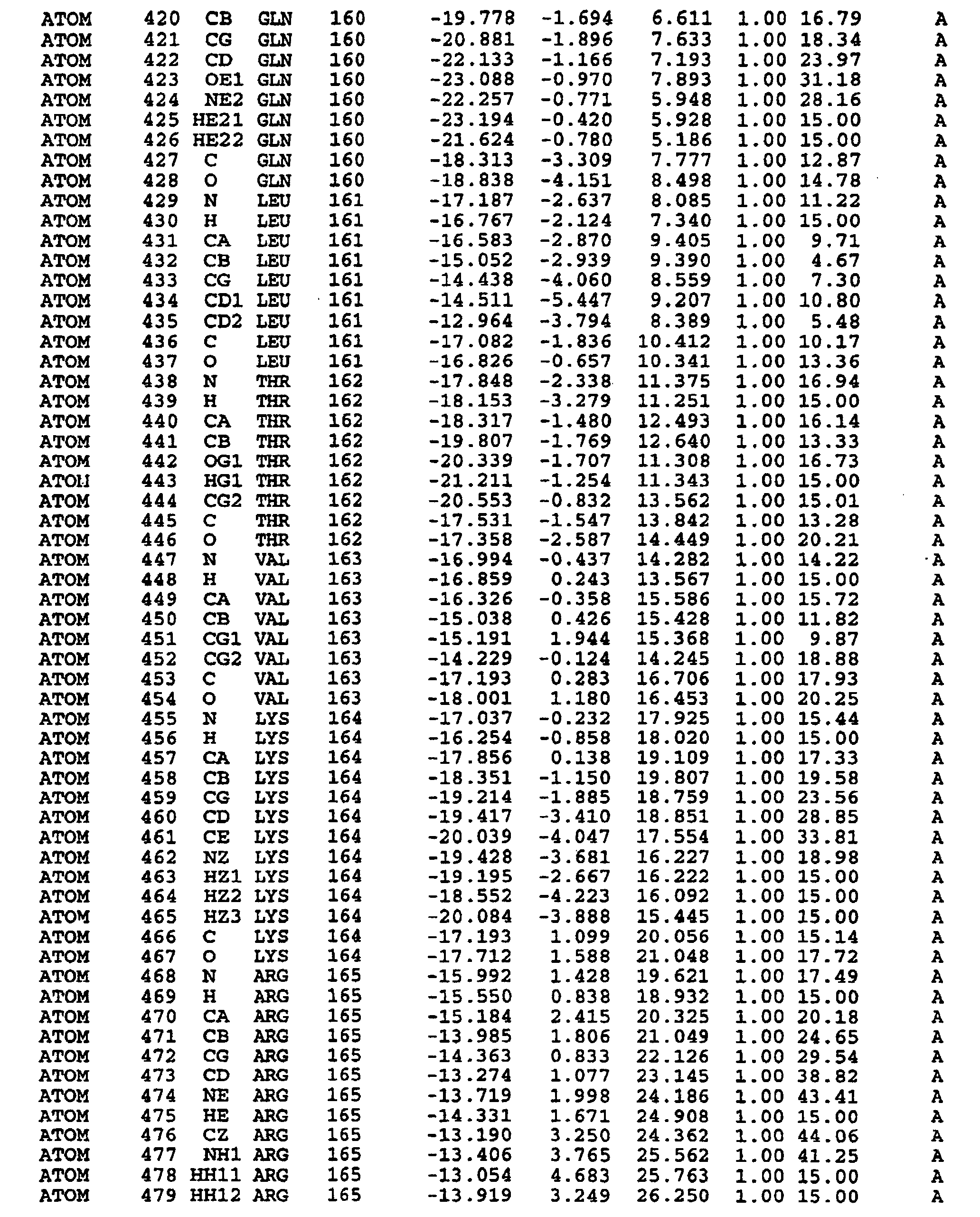

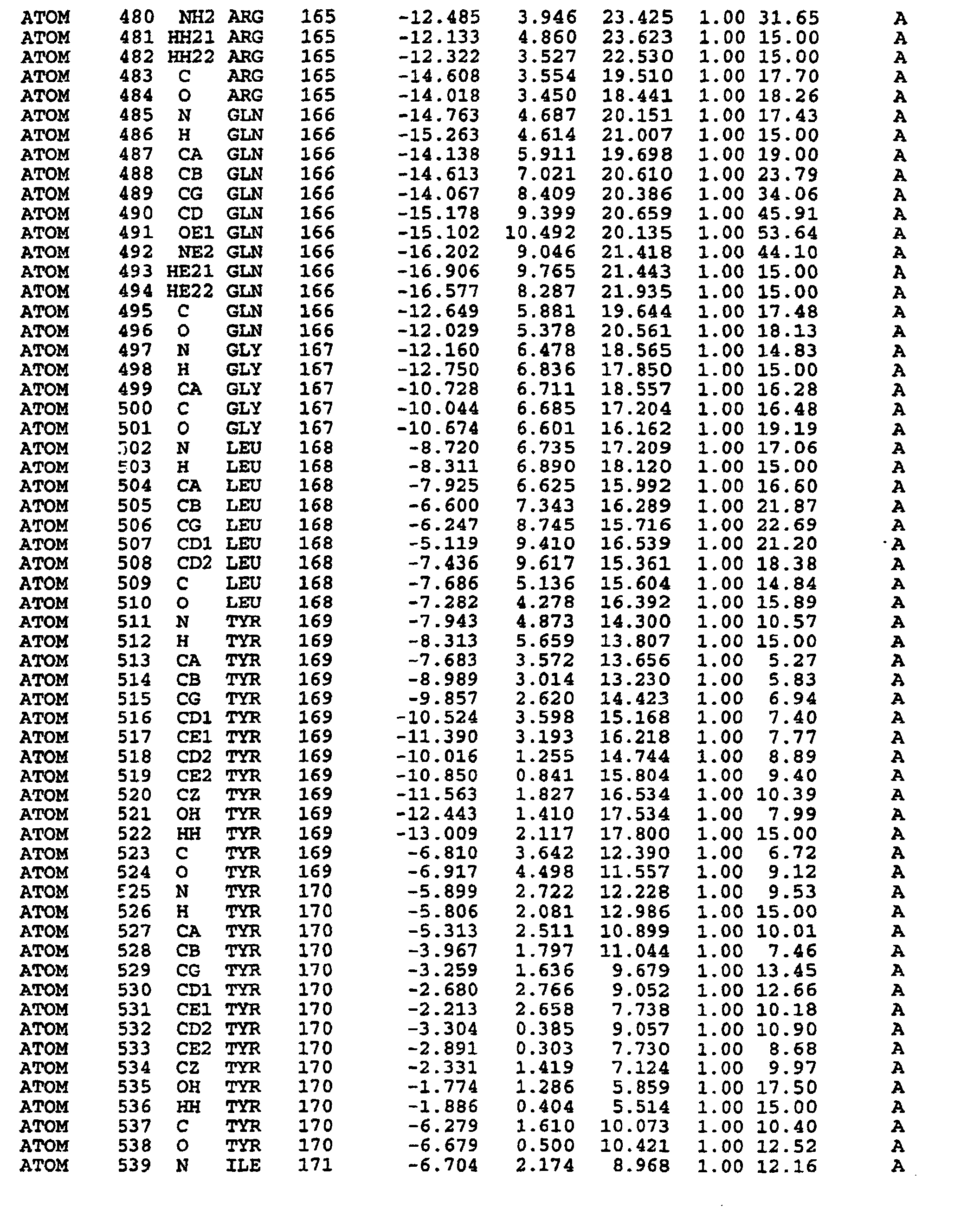

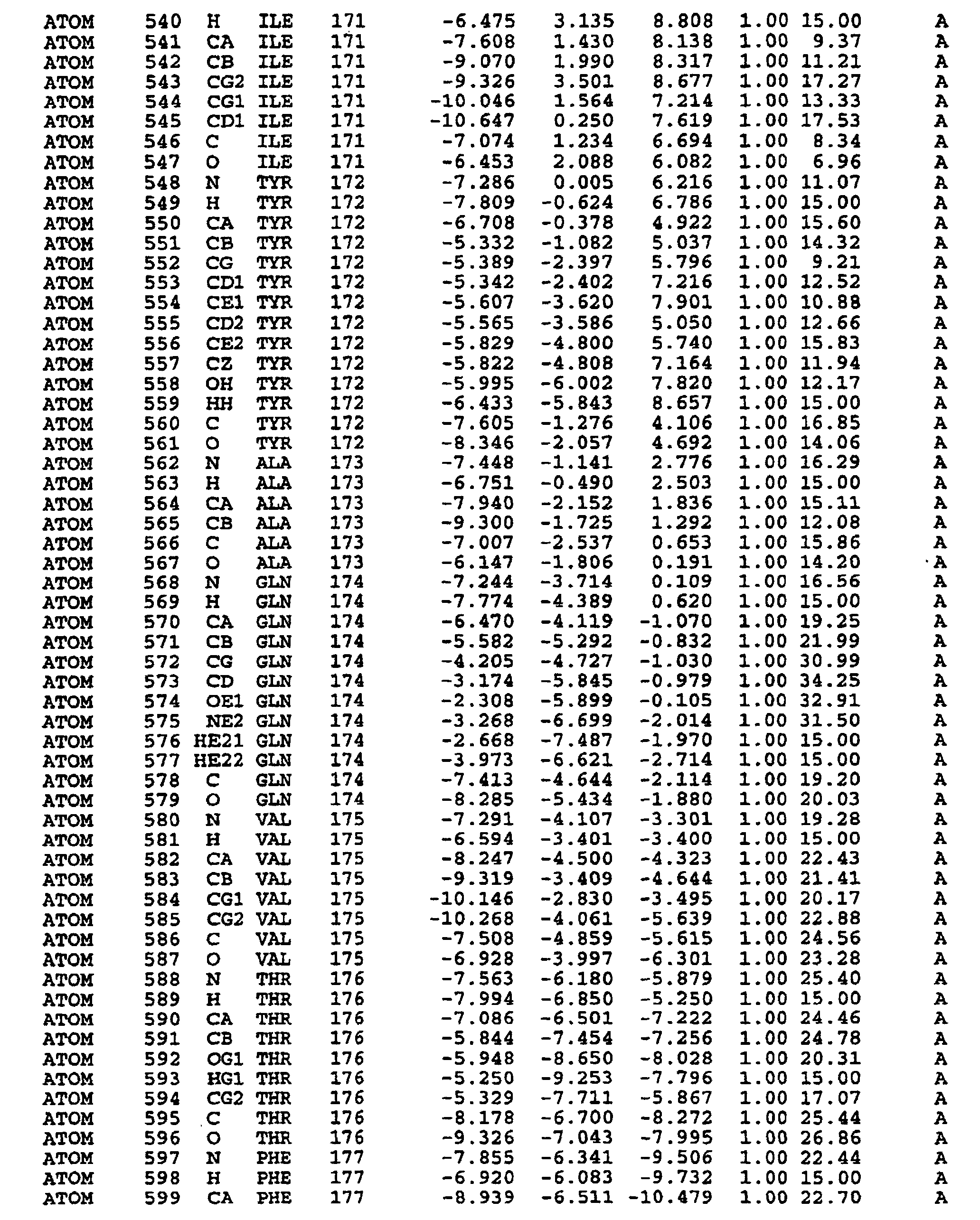

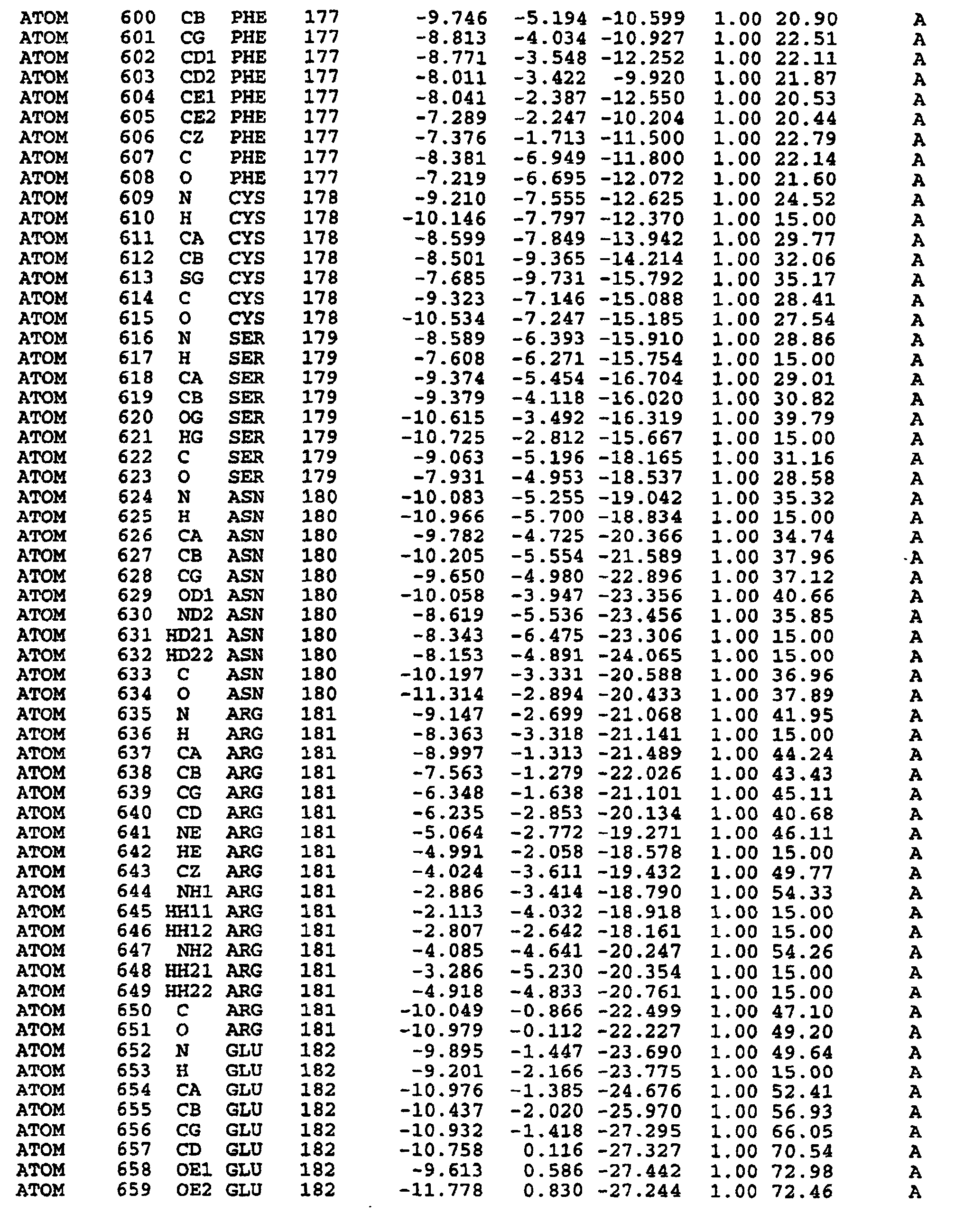

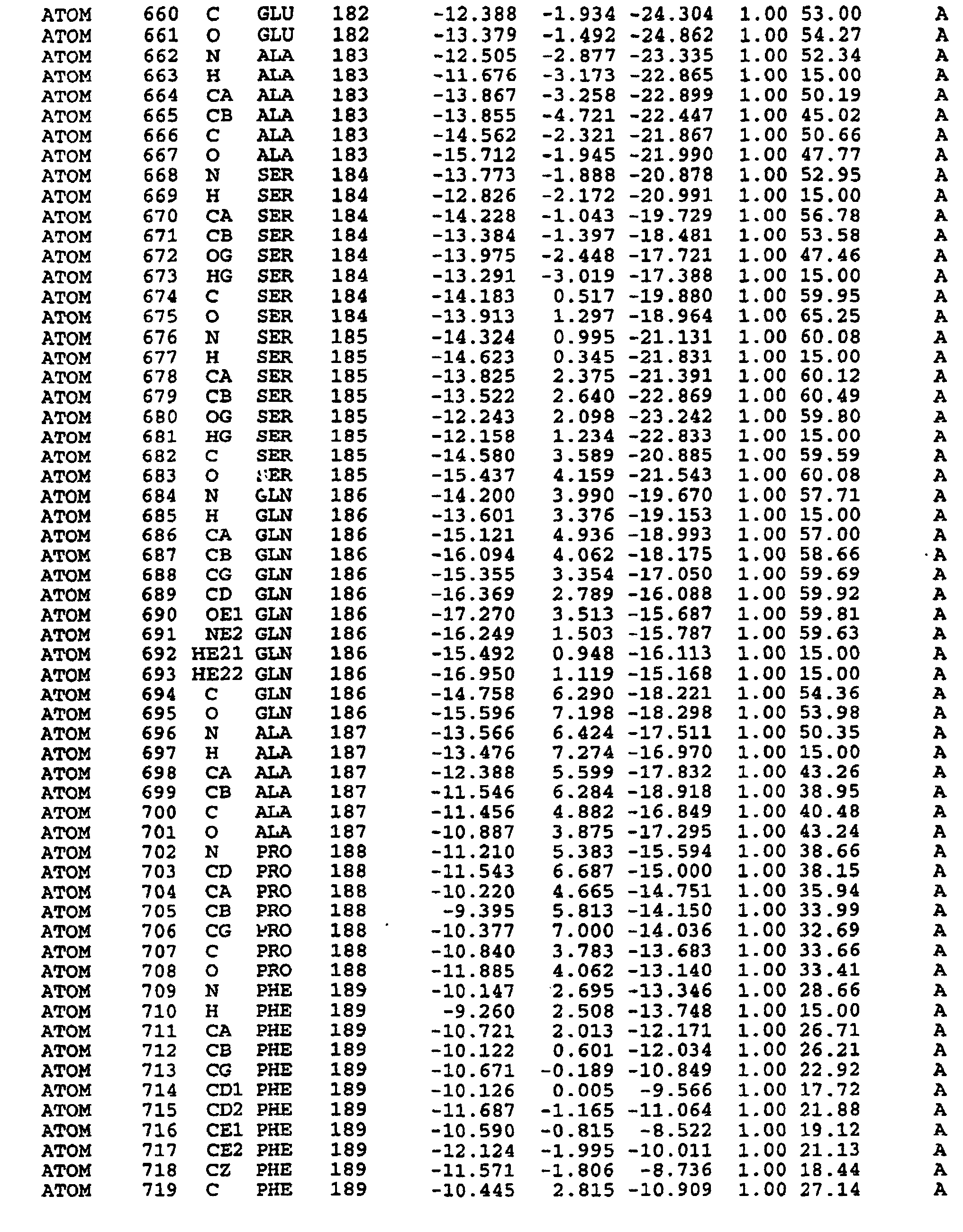

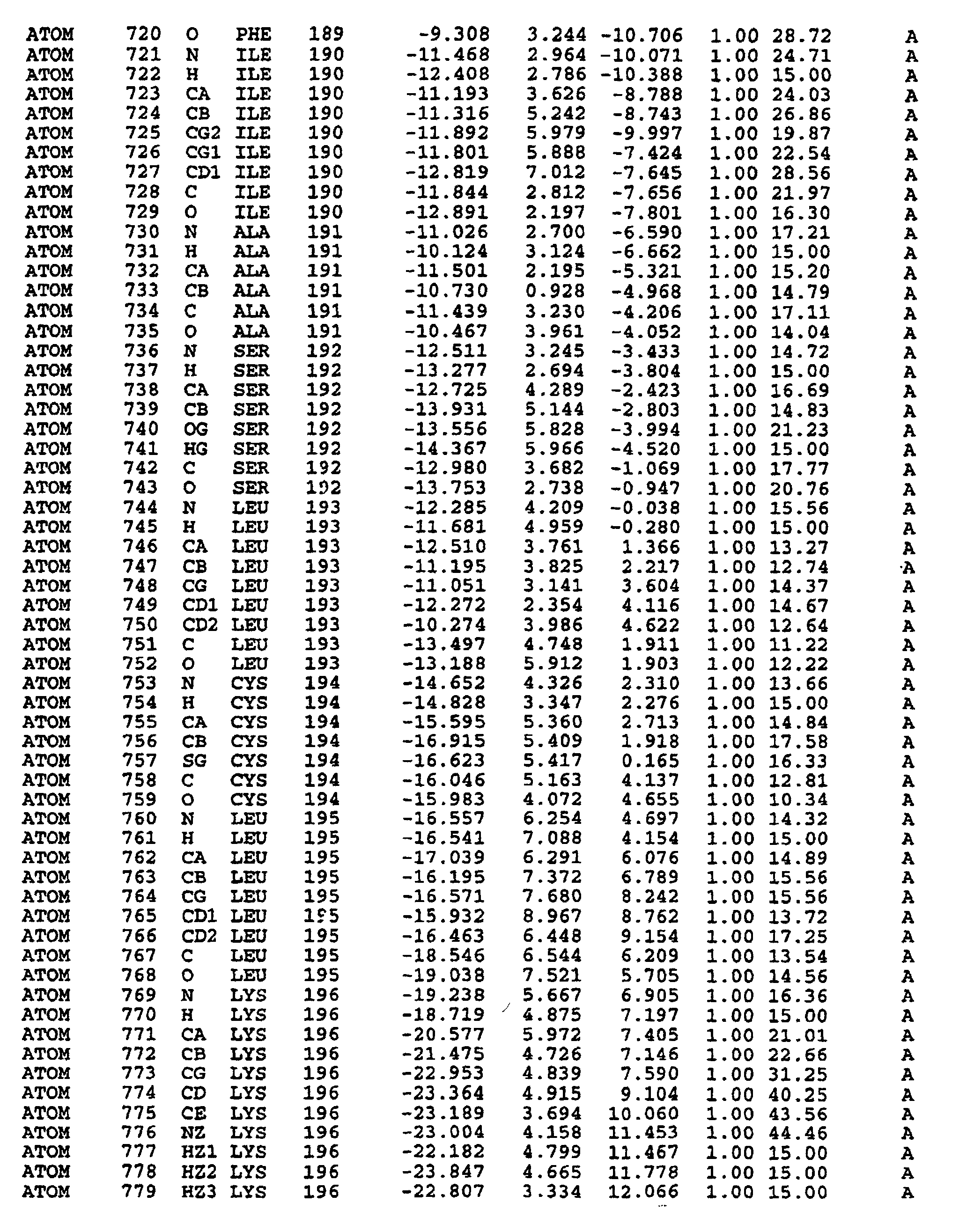

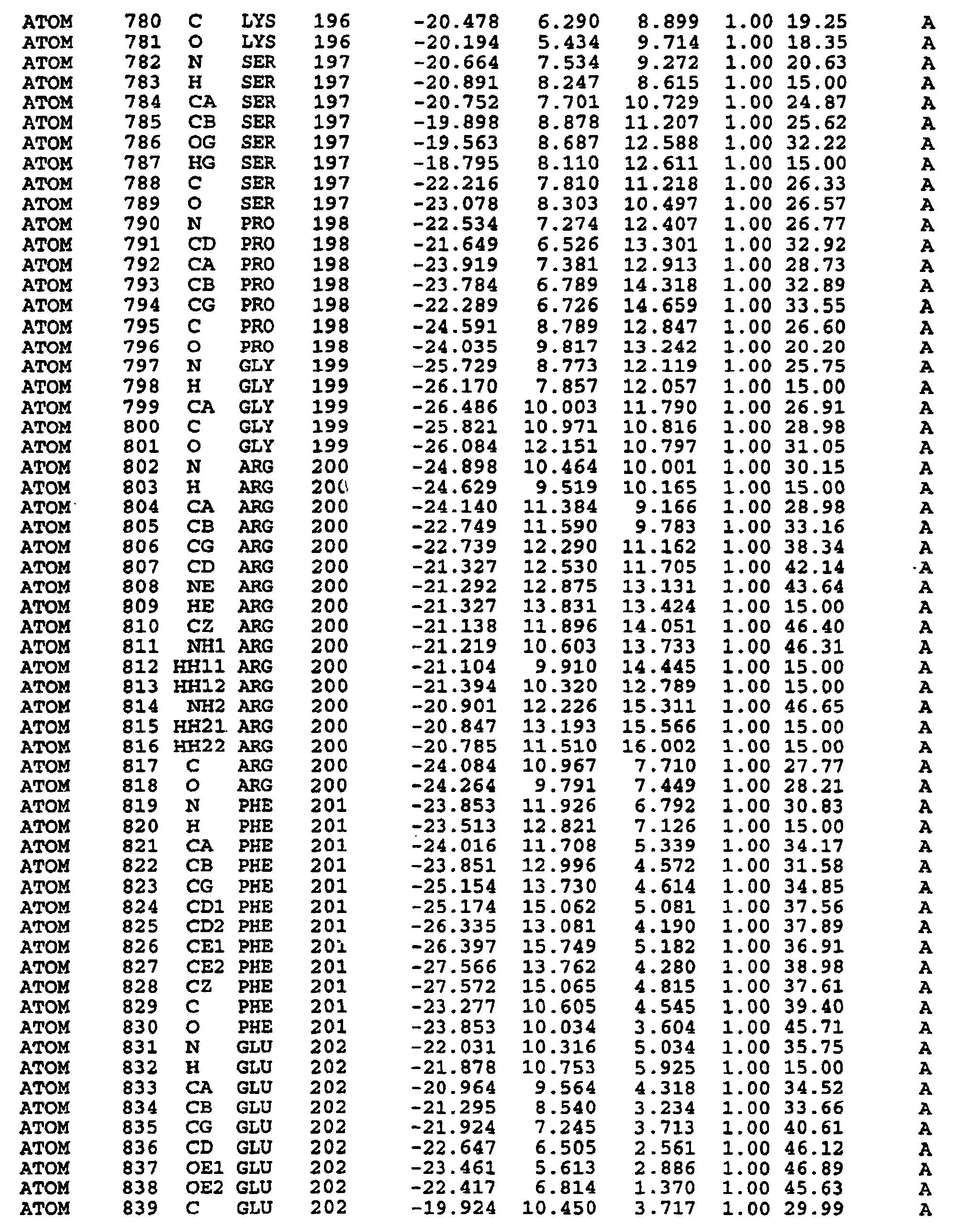

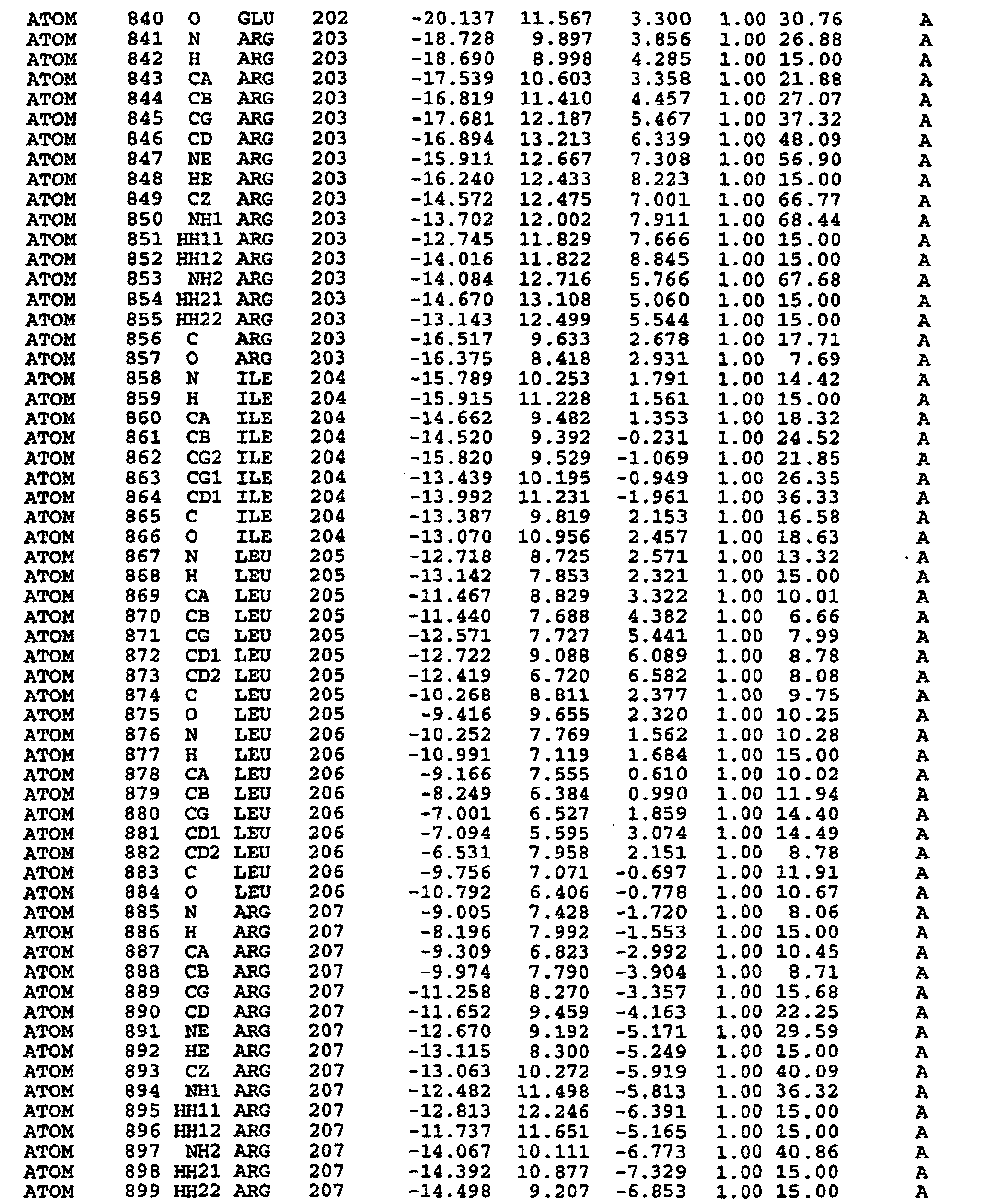

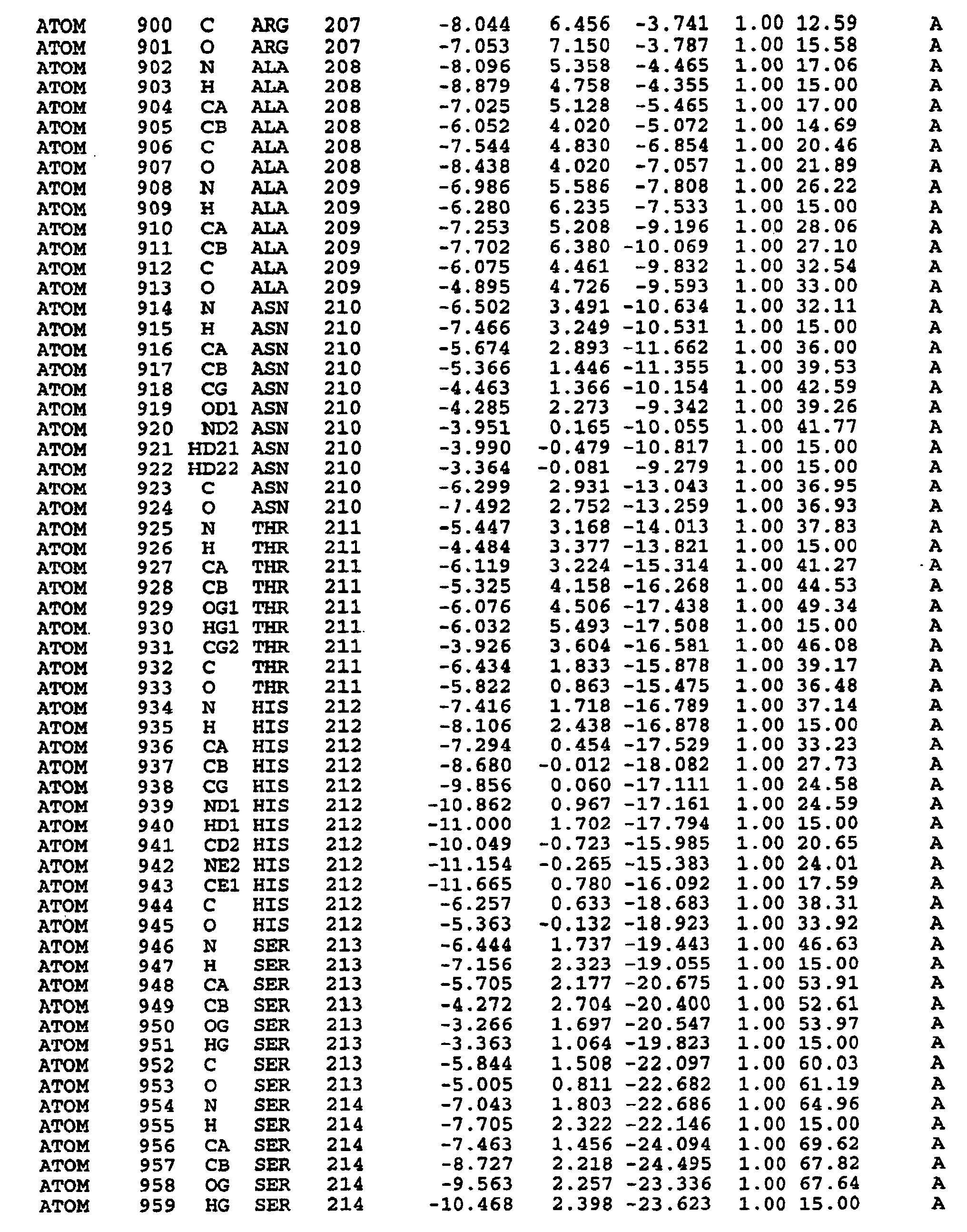

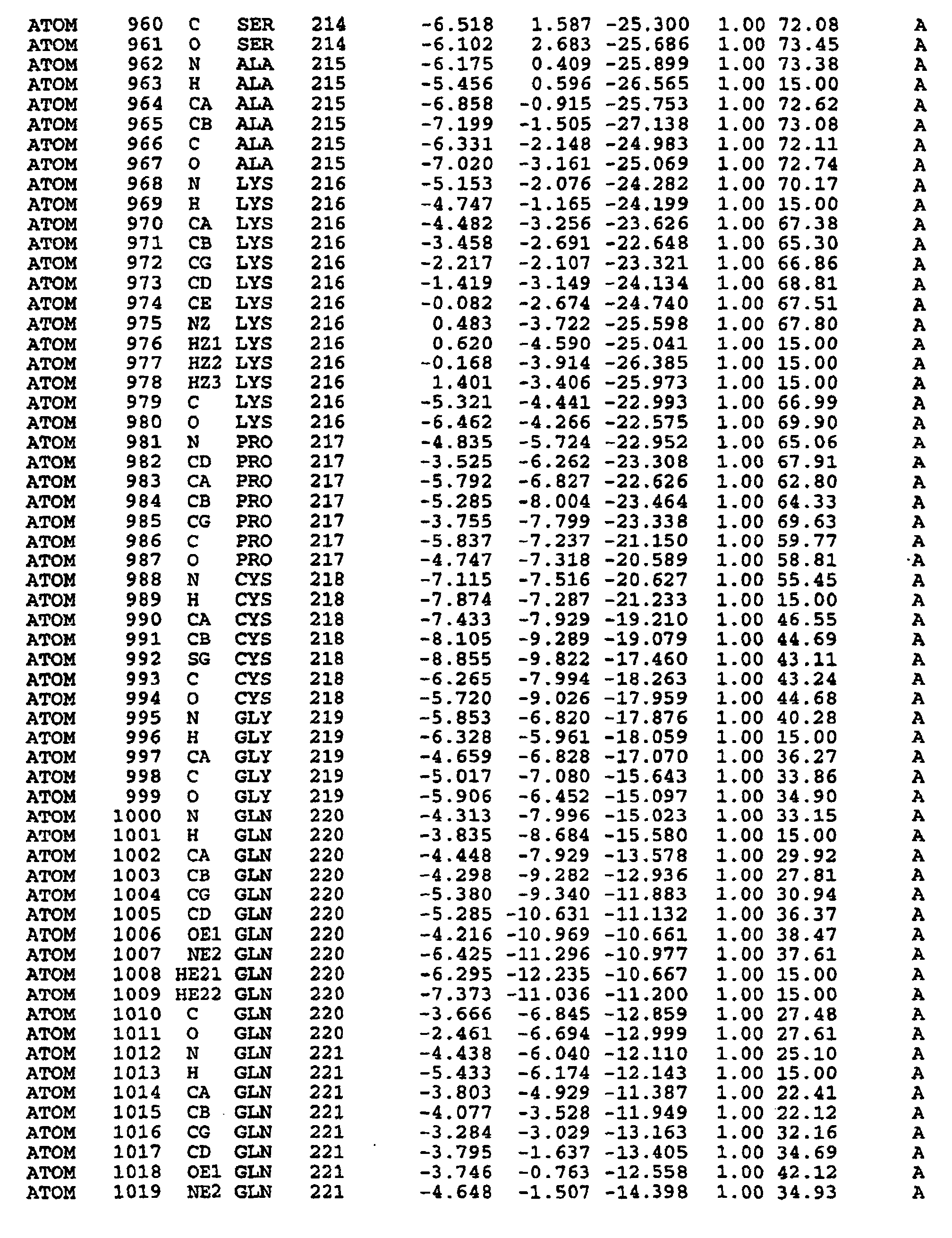

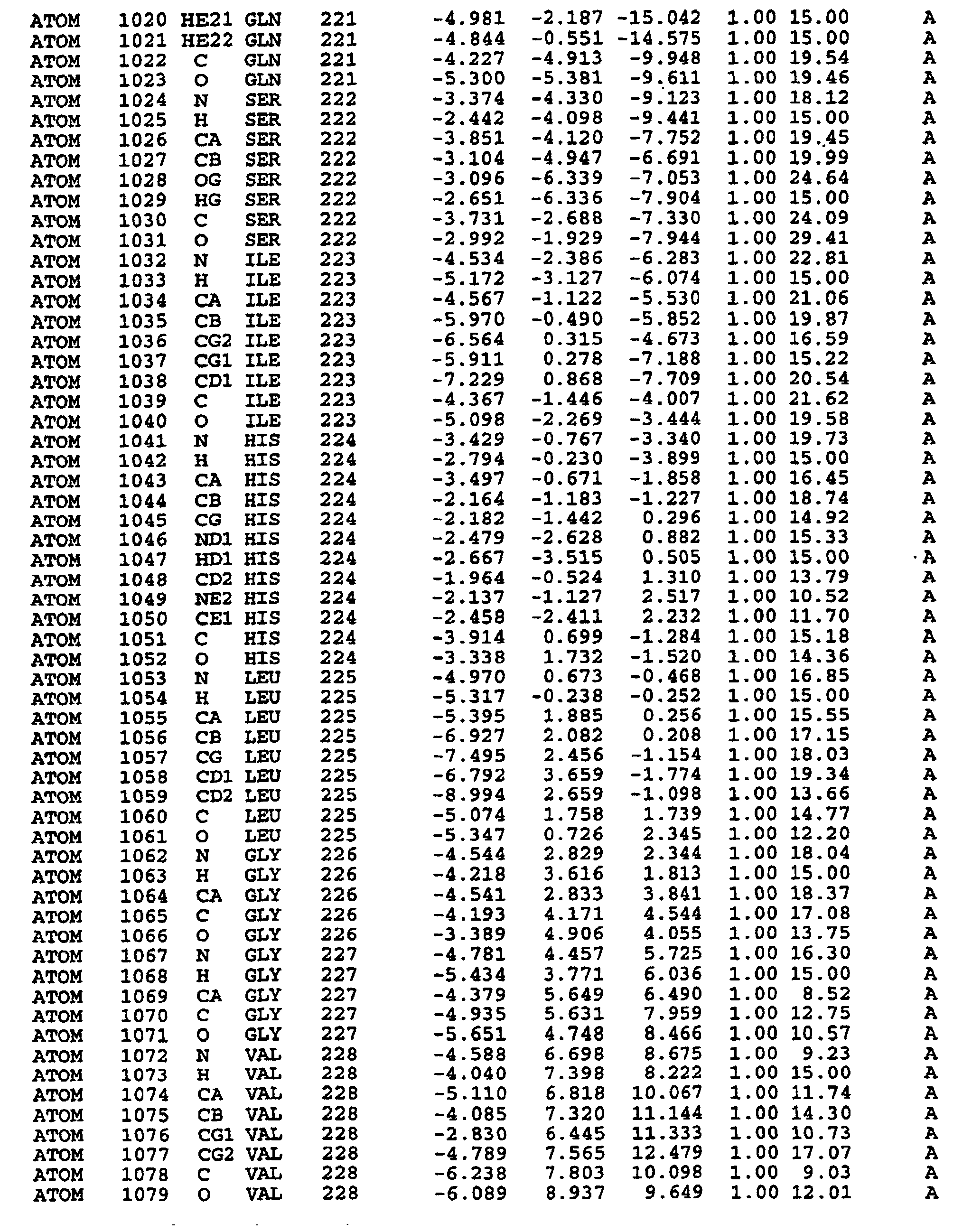

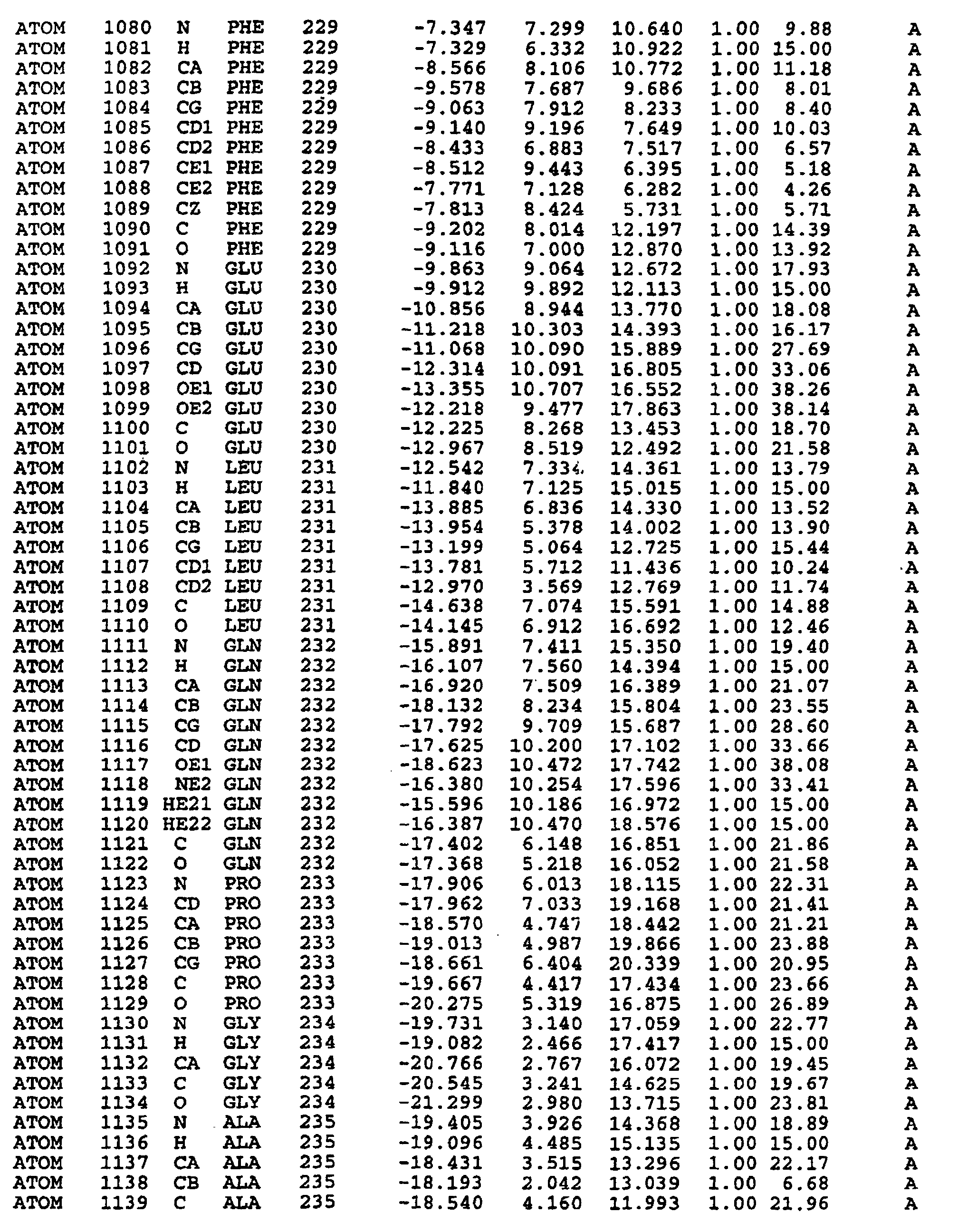

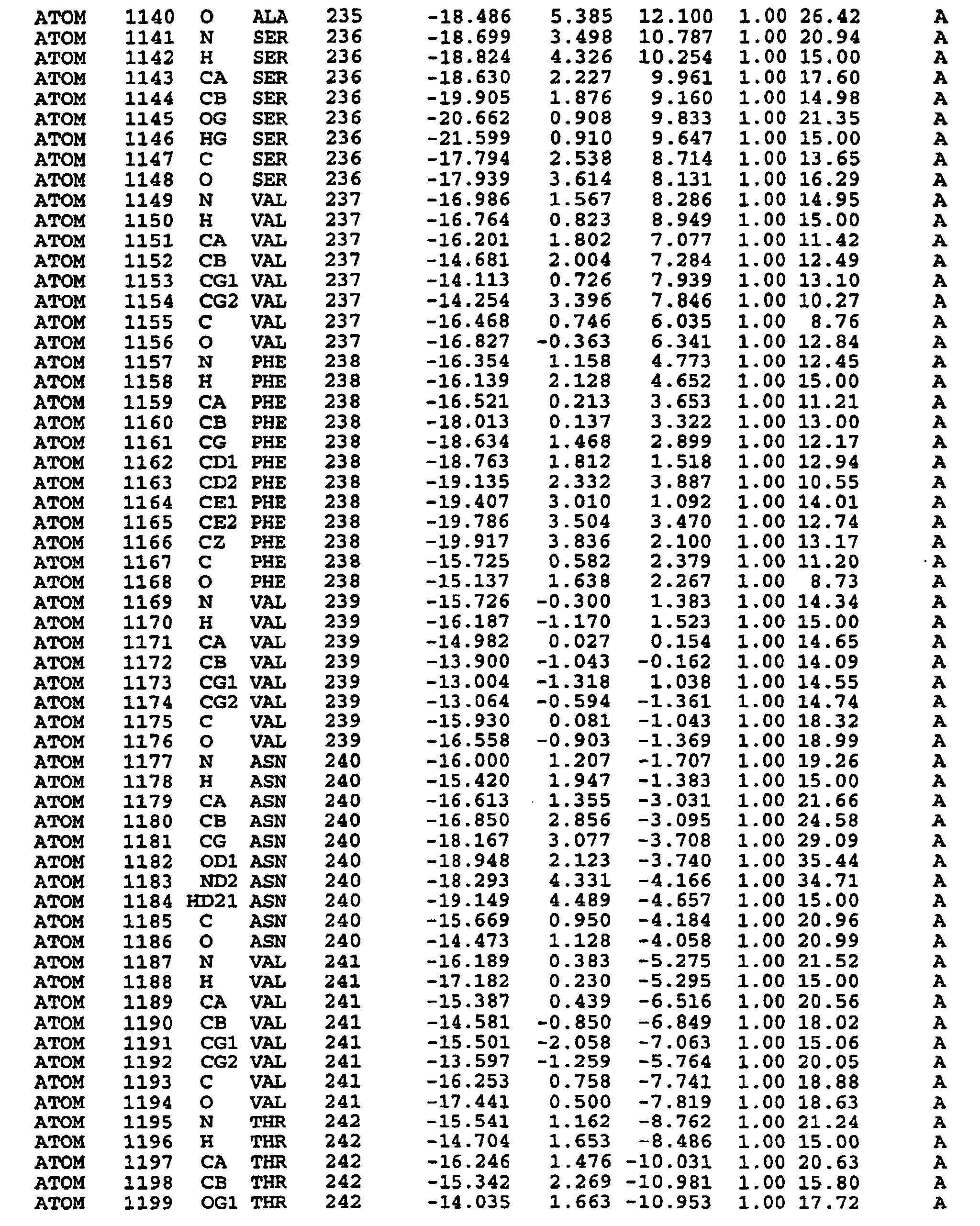

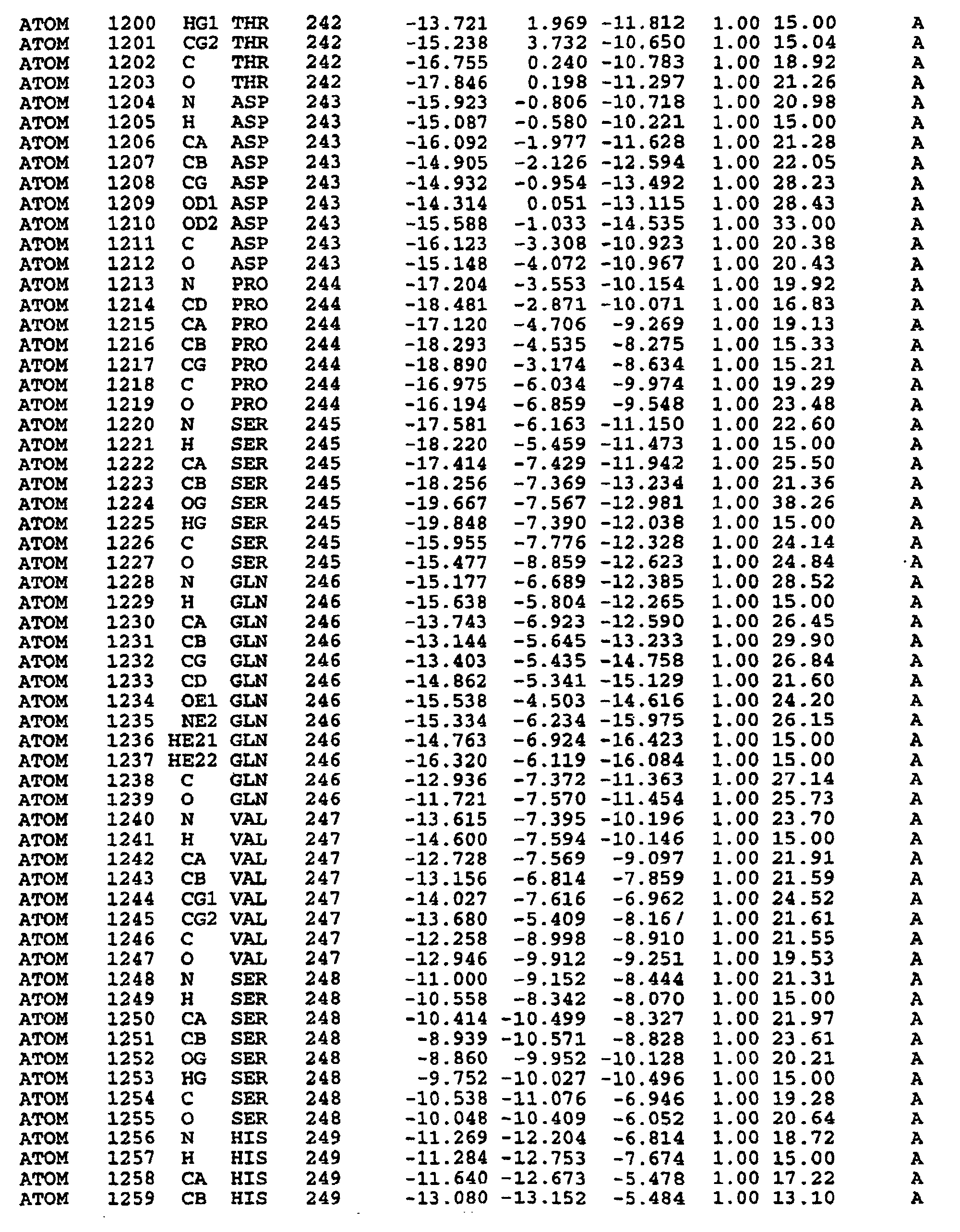

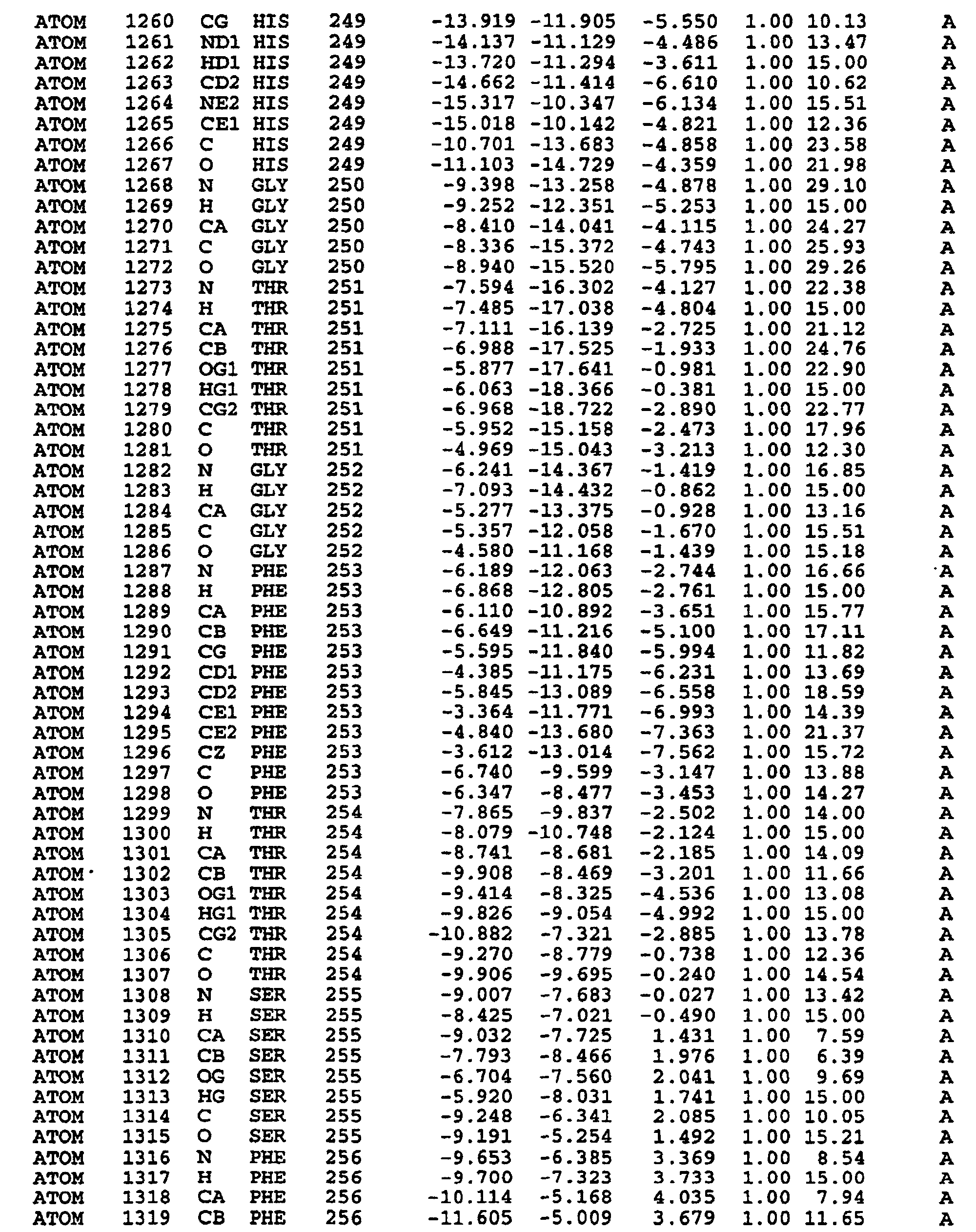

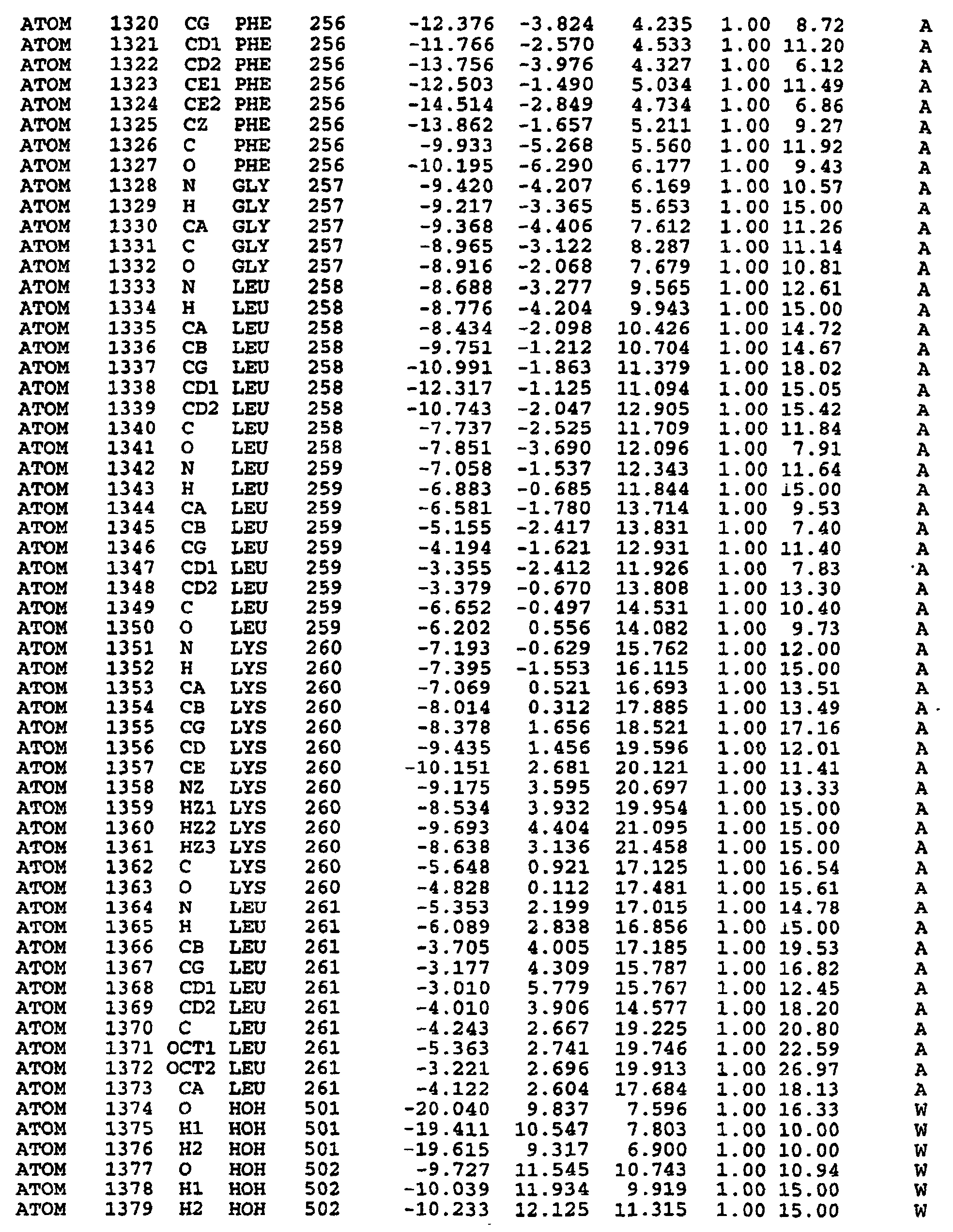

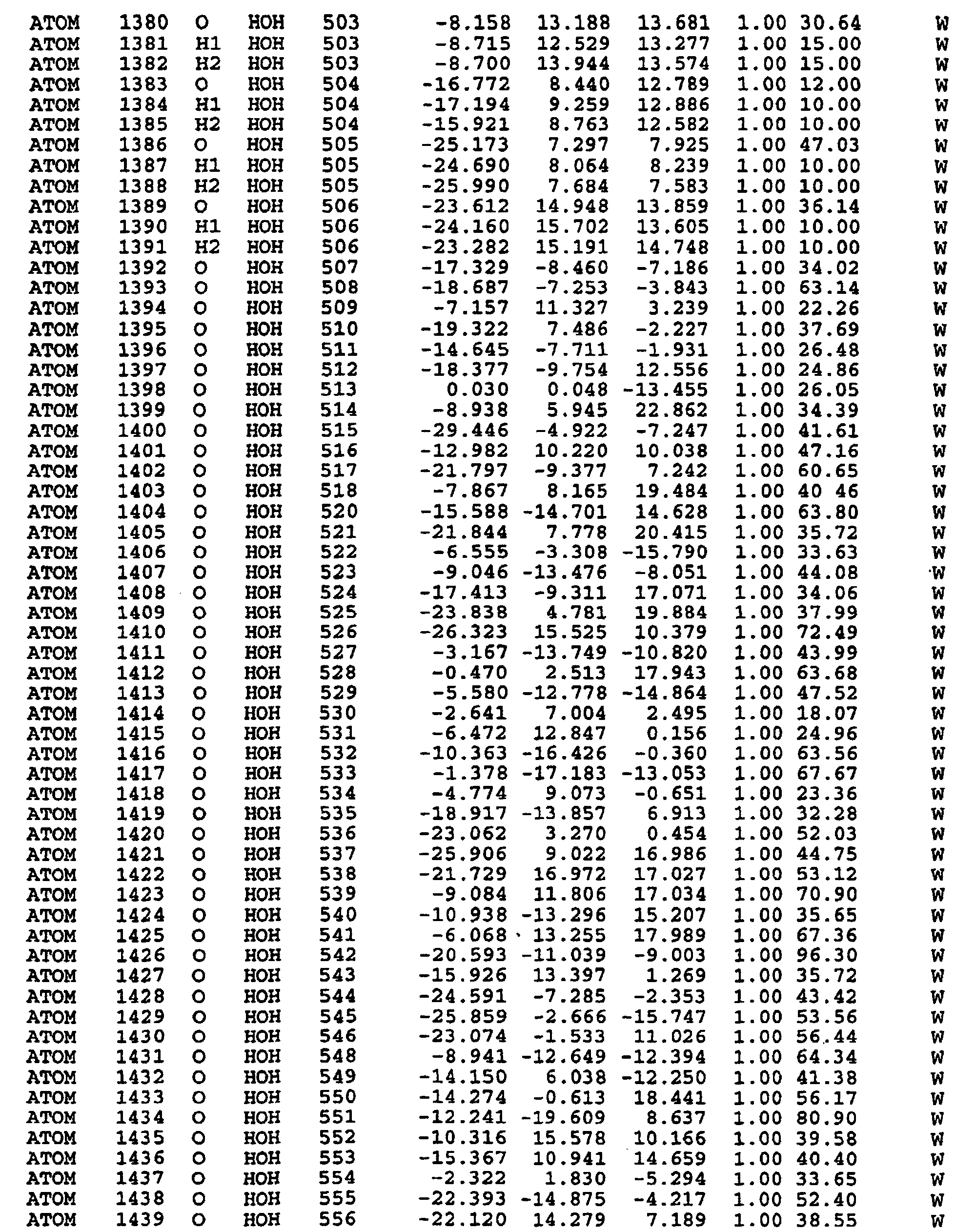

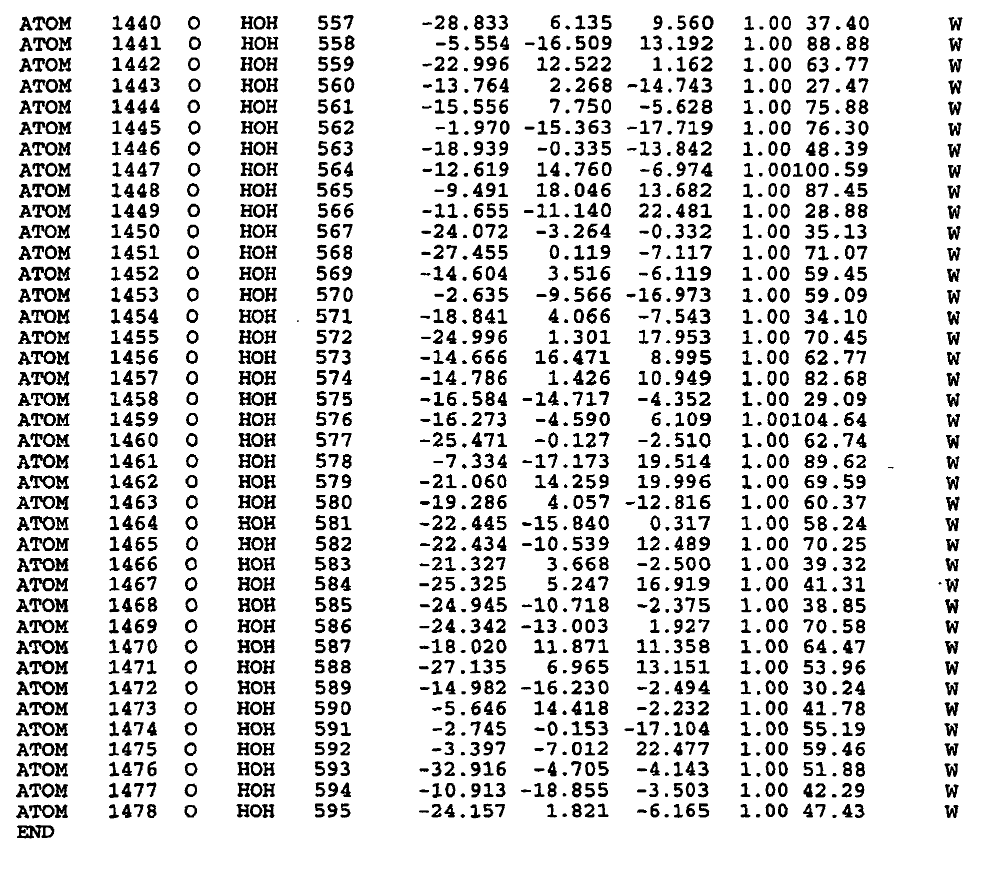

## Claims

1. A method of preparing a crystal of CD40 ligand comprising the steps of:
(a) providing an aqueous solution comprising a fragment of CD40 ligand;
(b) providing a reservoir solution comprising a precipitating agent;
(c) mixing a volume of said aqueous solution with a volume of said reservoir solution thereby forming a mixed volume; and
(d) crystallizing at least a portion of said mixed volume.

2. The method of claim 1 wherein the aqueous solution of CD40 ligand provided in step (a) has a concentration of CD40 ligand of about 1 to about 50 mg per ml.

3. The method of claim 2 wherein the aqueous solution has a concentration of CD40 iigand of about 5 mg per ml to about 15 mg per ml.

4. The method of claim 3 wherein the aqueous solution has a concentration of CD40 ligand of about 10 mg per ml.

5. The method of any one of claims 1 to 4 wherein the precipitating agent is selected from the group consisting of sodium citrate, ammonium sulfate and polyethylene glycol.

6. The method of any one of claims 1 to 5 wherein the concentration of the precipitating agent in the reservoir solution is about 1.0 M to about 1.5 M.

7. The method of claim 6 wherein the concentration of precipitating agent is about 1.2 M.

8. The method of any one of claims 1 to 7 wherein the pH of the reservoir solution is about 4 to about 10.

9. The method of claim 8 wherein the pH is about 7.5.

10. The method of any one of claims 1 to 9 wherein step (d) is by vapor diffusion crystallization, batch crystallization, liquid bridge crystallization or dialysis crystallization.

11. A crystal formed by a functional fragment of the extracellular domain of CD40 ligand having approximately the following cell constants: a+b=77.17 x 10⁻¹⁰ M (Å), c= 90.46 x 10⁻¹⁰ M (Å), α = β =90°, γ = 120°, and a space group of R3.

12. The crystal of CD40 ligand (116-261) of claim 11, or a homolog thereof, wherein said crystal comprises a binding site, said binding site comprising amino acids Lys143, Arg203, Arg207 and Tyr145.

13. The crystal of claim 11 or 12 or a homolog thereof wherein said crystal comprises Arg207 surrounded by at least two hydrophobic residues.

14. The crystal of claim 12 or 13 wherein said crystal further comprises at least 30 amino acids selected from the group consisting of: lle127, Ser128, Glu129, Ala130, Ser131, Thr135, Ser136, Ala141, Glu142, Gly144, Tyr146, Cys178, Asn180, Ser185, Gln186, Ala187, Pro188, lle190, Ala191, Ser192, Ser197, Pro198, Gly199, Arg200, Phe201, Glu202, lle204, Ala209, Thr211, Pro217, Cys218, Gly219, Gln220, Glu230, Leu231, Gln232, Asn240, Val241, Thr242, Asp243, Ser245, Val247, Ser248, His249, Gly250, Thr251, Gly252 and Phe253.

15. A method for determining at least a portion of a three dimensional structure of a molecular complex, said complex comprising at least a fragment of CD40 ligand, said method comprising the steps of:
(a) determining the structural coordinates of a crystal of the fragment of CD40 ligand;
(b) calculating phases from the structural coordinates;
(c) calculating an electron density map from the phases obtained in step (b);
(d) determining the structure of at least a portion of the complex based upon said electron density map.

16. The method of claim 15 wherein the structural coordinates used in step (a) are the same as or deviate by less than about 2Å from those described in Table 1.

17. A. method for evaluating the ability of a chemical entity to associate with CD40 ligand or CD40, a fragment of CD40 or CD40 ligand, or a complex comprising CD40 ligand, CD40, or homologs thereof, said method comprising the steps of:
(a) employing computational or experimental means to perform a fitting operation between the chemical entity and said CD40 ligand or CD40, fragment or complex thereof, thereby obtaining data related to said association; and
(b) analyzing the data obtained in step (a) to determine the characteristics of the association between the chemical entity and said CD40 ligand or CD40, fragment or complex.

18. The method of claim 17 further comprising identifying, **characterizing** or designing a chemical entity having a desired association with a CD40 ligand, or fragment thereof by using the structural coordinates of a crystal of CD40 ligand of any one of claims 11 to 14.

19. The method of claim 18 further comprising the step of optimizing the binding characteristics of the chemical entity identified, **characterized** or designed.

20. The method of claim 19 further comprising the step of determining the orientation of ligands in a binding site of CD40 ligand.

21. A heavy atom derivative of a crystallized form of CD40 ligand.

22. A heavy atom derivative of the crystal of any one of claims 11 to 14.

23. Use of the structural coordinates of CD40 ligand, or portions thereof, to solve a crystal form of a mutant homologue or co-complex of CD40 ligand or a fragment thereof by molecular replacement.

24. Use of a crystal of the CD40 ligand of any one of claims 11 to 14 or the structural coordinates thereof for computationally or experimentally evaluating a chemical entity to obtain information about its association with the binding site of the CD40 ligand.

25. The use of claim 24 wherein the crystal has the structural coordinates described in Table 1.

26. The use of claim 24 wherein said crystal is the same as the crystal of CD40 ligand described by the coordinates in Table 1 or by coordinates deviating by less than about 2Å from the coordinates in Table 1.

27. Use of a CD40 ligand crystal of any one of claims 11 to 14 to determine binding interactions between a chemical entity and CD40 ligand.

28. A machine readable data storage medium comprising a data storage material encoded with machine readable data which, when read by an appropriate machine, is capable of displaying a three dimensional representation of a crystal of a molecule or molecular complex comprising a fragment of CD40L having a binding site comprising amino acids Lys143, Arg203, Arg207 and Tyr145.

29. A method for the production of a pharmaceutical composition comprising the method of any one of claims 17 to 20 and furthermore formulating the chemical entity identified in the last step of the method of any one of claims 17 to 20 in a pharmaceutically acceptable form.

## Patentansprüche

1. Verfahren zur Herstellung eines Kristalls eines CD40-Liganden, umfassend die Schritte:
(a) Bereitstellen einer wässrigen Lösung, die ein Fragment eines CD40-Ligangen umfasst;
(b) Bereitstellen einer Reservoir-Lösung, die ein präzipitierendes Agens umfasst;
(c) Mischen eines Volumens der wässrigen Lösung mit einem Volumen der Reservoir-Lösung, wodurch ein gemischtes Volumen gebildet wird; und
(d) Kristallisieren von zumindest einem Teil des gemischten Volumens.

2. Verfahren nach Anspruch 1, wobei die wässrige Lösung eines CD40-Liganden, die in Schritt (a) bereitgestellt wird, eine CD40-Liganden-Konzentration von etwa 1 bis etwa 50 mg pro ml aufweist.

3. Verfahren nach Anspruch 2, wobei die wässrige Lösung eine CD40-Liganden-Konzentration von etwa 5 mg pro ml bis etwa 15 mg pro ml aufweist.

4. Verfahren nach Anspruch 3, wobei die wässrige Lösung eine CD40-Liganden-Konzentration von etwa 10 mg pro ml aufweist.

5. Verfahren nach einem der Ansprüche 1 bis 4, wobei das präzipitierende Agens ausgewählt ist aus der Gruppe bestehend aus Natriumcitrat, Ammoniumsulfat und Polyethylenglykol.

6. Verfahren nach einem der Ansprüche 1 bis 5, wobei die Konzentration des präzipitierenden Agens in der Reservoir-Lösung etwa 1,0 M bis etwa 1,5 M ist.

7. Verfahren nach Anspruch 6, wobei die Konzentration des präzipitierenden Agens etwa 1,2 M ist.

8. Verfahren nach einem der Ansprüche 1 bis 7, wobei der pH-Wert der Reservoir-Lösung etwa 4 bis etwa 10 ist.

9. Verfahren nach Anspruch 8, wobei der pH-Wert etwa 7,5 ist.

10. Verfahren nach einem der Ansprüche 1 bis 9, wobei Schritt (5) durch Dampfdiffusionskristallisation, Batch-Kristallisation, Flüssigbrücken-Kristallisation oder Dialyse-Kristallisation erfolgt.

11. Kristall, der von einem funktionellen Fragment der extrazellulären Domäne des CD40-Liganden gebildet wird, der ungefähr die folgenden Zellkonstanten aufweist: a+b = 77,17 x 10⁻¹⁰ M (Å), c = 90,46 x 10¹⁰ M (Å), α = β = 90°, γ = 120° und Raumgruppe R3.

12. Kristall des CD40-Liganden (116-261) nach Anspruch 11 oder ein Homolog davon, wobei der Kristall eine Bindungsstelle umfasst, welche die Aminosäuren Lys143, Arg203, Arg207 und Tyr145 umfasst.

13. Kristall nach Anspruch 11 oder 12 oder ein Homolog davon, wobei der Kristall Arg207 umfasst, das von mindestens zwei hydrophoben Resten umgeben ist.

14. Kristall nach Anspruch 12 oder 13, wobei der Kristall weiterhin zumindest 30 Aminosäuren umfasst, die ausgewählt sind aus der Gruppe bestehend aus: Ile127, Ser128, Glu129, Ala130, Ser131, Thr135, Ser136, Ala141, Glu142, Gly144, Tyr146, Cys178, Asn180, Ser185, Gln186, Ala187, Pro188, Ile190, Ala191, Ser192, Ser197, Pro198, Gly199, Arg200, Phe201, Glu202, Ile204, Ala209, Thr211, Pro217, Cys218, Gly219, Gln220, Glu230, Leu231, Gln232, Asn240, Val241, Thr242, Asp243, Ser245, Val247, Ser248, His249, Gly250, Thr251, Gly252 and Phe253.

15. Verfahren zum Bestimmen von zumindest einem Teil einer dreidimensionalen Struktur eines molekularen Komplexes, wobei der Komplex zumindest ein Fragment eines CD40-Liganden umfasst, wobei das Verfahren die Schritte umfasst:
(a) Bestimmen der Strukturkoordinaten eines Kristalls des Fragments eines CD40-Liganden;
(b) Berechnen der Phasen aus den Strukturkoordinaten;
(c) Berechnen einer Elektronendichtekarte aus den Phasen, die in Schritt (b) erhalten wurden;
(d) Bestimmen der Struktur von zumindest einem Teil des Komplexes auf der Basis der Elektronendichtekarte.

16. Verfahren nach Anspruch 15, wobei die Strukturkoordinaten, die in Schritt (a) verwendet werden, dieselben sind wie die in Tabelle 1 beschriebenen oder um weniger als etwa 2 Å von den in Tabelle 1 beschriebenen abweichen.

17. Verfahren zur Evaluierung der Fähigkeit einer chemischen Einheit zur Assoziation mit einem CD40-Ligand oder CD40, mit einem Fragment von CD40 oder einem CD40-Ligand oder mit einem Komplex, der einen CD40-Ligand, CD40 oder Homologe davon umfasst, wobei das Verfahren die Schritte umfasst:
(a) Anwenden von rechnerischen oder experimentellen Mitteln, um eine Zusammenpassungsüberprüfung zwischen der chemischen Einheit und dem CD40-Ligand oder CD40, einem Fragment oder einem Komplex davon auszuführen, wodurch Daten erhalten werden, die mit der Assoziation zusammenhängen; und
(b) Analysieren der Daten, die in Schritt (a) erhalten wurden, um die Merkmale der Assoziation zwischen der chemischen Einheit und dem CD40-Ligand oder CD40, dem Fragment oder Komplex zu bestimmen.

18. Verfahen nach Anspruch 17, das weiterhin das Identifizieren, Charakterisieren oder Entwerfen einer chemischen Einheit umfasst, die eine gewünschte Assoziation mit einem CD40-Ligand oder Fragment davon aufweist, indem die Strukturkoordinaten eines Kristalls eines CD40-Liganden nach einem der Ansprüche 11 bis 14 verwendet werden.

19. Verfahren nach Anspruch 18, das weiterhin den Schritt des Optimierens der Bindungseigenschaften der identifizierten, charakterisierten oder entworfenen chemischen Einheit umfasst.

20. Verfahren nach Anspruch 19, das weiterhin den Schritt des Bestimmens der Orientierung von Liganden in einer Bindungsstelle des CD40-Liganden umfasst.

21. Schweratomderivat einer kristallisierten Form des CD40-Liganden.

22. Schweratomderivat des Kristalls nach einem der Ansprüche 11 bis 14.

23. Verwendung der Strukturkoordinaten eines CD40-Liganden oder Teilen davon, um eine Kristallform eines mutanten Homologen oder eines Co-Komplexes eines CD40-Liganden oder eines Fragments davon durch molecular replacement aufzulösen.

24. Verwendung eines Kristalls des CD40-Liganden nach einem der Ansprüche 11 bis 14 oder der Strukturkoordinaten davon für das rechnerische oder experimentelle Evaluieren einer chemischen Einheit, um Informationen über ihre Assoziation mit der Bindungsstelle des CD40-Liganden zu erhalten.

25. Verwendung nach Anspruch 24, wobei der Kristall die in Tabelle 1 beschriebenen Strukturkoordinaten aufweist.

26. Verwendung nach Anspruch 24, wobei der Kristall der gleiche ist wie der Kristall eines CD40-Liganden, der durch die Koordinaten in Tabelle 1 oder durch Koordinaten, die um weniger als etwa 2 Å von den Koordinaten in Tabelle 1 abweichen, beschrieben ist.

27. Verwendung eines CD40-Liganden-Kristalls nach einem der Ansprüche 11 bis 14, um die Bindungswechselwirkungen zwischen einer chemischen Einheit und einem CD40-Liganden zu bestimmen.

28. Maschinenlesbares Datenspeichermedium, umfassend ein Datenspeichermaterial, das mit maschinenlesbaren Daten codiert ist, das, wenn es von einer geeigneten Maschine gelesen wird, in der Lage ist, eine dreidimensionale Wiedergabe eines Kristalls eines Moleküls oder molekularen Komplexes darzustellen, das/der ein Fragment eines CD40L umfasst, das eine Bindungsstelle aufweist, die die Aminosäuren Lys143, Arg203, Arg207 und Tyr145 umfasst.

29. Verfahren zur Herstellung eines Arzneimittels, umfassend das Verfahren nach einem der Ansprüche 17 bis 20 und weiterhin das Formulieren der chemischen Einheit, die im letzten Schritt des Verfahrens nach einem der Ansprüche 17 bis 20 identifiziert wurde, in eine pharmazeutisch verträgliche Form.

## Revendications

1. Méthode de préparation d'un cristal d'un ligand de CD40 comprenant les étapes de :
(a) prévoir une solution aqueuse comprenant un fragment d'un ligand de CD40;
(b) prévoir une solution en réservoir comprenant un agent précipitant;
(c) mélanger un volume de ladite solution aqueuse avec un volume de ladite solution en réservoir pour ainsi former un volume mélangé; et
(d) cristalliser au moins une portion dudit volume mélangé.

2. Méthode de la revendication 1, où la solution aqueuse du ligand de CD40 prévue à l'étape (a) a une concentration du ligand de CD40 d'environ 1 à environ 60 mg par ml.

3. Méthode de la revendication 2, où la solution aqueuse a une concentration du ligand de CD40 d'environ 5 mg par ml à environ 15 mg par ml.

4. Méthode de la revendication 3, où la solution aqueuse a une concentration du ligand de CD40 d'environ 10 mg par ml .

5. Méthode de l'une quelconque des revendications 1 à 4, où l'agent précipitant est sélectionné dans le groupe consistant en citrate de sodium, sulfate d'ammonium et polyéthylène glycol.

6. Méthode de l'une quelconque des revendications 1 à 5, où la concentration de l'agent précipitant dans la solution en réservoir est d'environ 1,0 M à environ 1,5 M.

7. Méthode de la revendication 6, où la concentration de l'agent précipitant est d'environ 1,2 M.

8. Méthode de l'une quelconque des revendications 1 à 7, où le pH de la solution en réservoir est d'environ 4 à environ 10.

9. Méthode de la revendication 8, où le pH est d'environ 7,5.

10. Méthode de l'une quelconque des revendications 1 à 9, où l'étape (d) est par cristallisation par diffusion en phase vapeur, cristallisation discontinue, cristallisation à pont liquide ou cristallisation par dialyse.

11. Cristal formé par un fragment fonctionnel du domaine extracellulaire d'un ligand de CD40 ayant à peu près les constantes cellulaires suivantes: a+b=77,17 x 10⁻¹⁰ M (Å) , c=90,46 x 10⁻¹⁰ M (Å), α=β=90°, γ= 120°, et un groupe spatial de R3 .

12. Cristal du ligand de CD40 (116-261) de la revendication 11 ou son homologue, où ledit cristal comprend un site de liaison, ledit site de liaison comprenant les acides aminés Lys143, Arg203, Arg207 et Tyr145.

13. Cristal de la revendication 11 ou 12 ou son homologue, où ledit cristal comprend Arg207 entouré d'au moins deux résidus hydrophobes.

14. Cristal de la revendication 12 ou 13, où ledit cristal comprend de plus au moins 30 acides aminés sélectionnés dans le groupe consistant en: Ile127, Ser128, Glu129, Ala130, Ser131, Thr135, Ser136, Ala141, Glu142, Gly144, Tyr146, Cys178, Asn180, Ser185, Gln186, Ala187, Pro188, Ile190, Ala191, Ser 192, Ser 197, Pro198, Gly199, Arg200, Phe201, Glu202, Ile204, Ala209, Thr211, Pro217, Cys218, Gly219, Gln220, Glu230, Leu231, Gln232, Asn240, Val241, Thr242, Asp243, Ser245, Val247, Ser248, His249, Gly250, Thr251, Gly252, et Phe253.

15. Méthode pour déterminer au moins une portion d'une structure tridimensionnelle d'un complexe moléculaire, ledit complexe comprenant au moins sur fragment d'un ligand de CD40, ladite méthode comprenant les étapes de:
(a) déterminer les coordonnées de structure d'un cristal du fragment du ligand de CD40;
(b) calculer les phases à partir des coordonnées de structure;
(c) calculer une carte de densité des électrons à partir des phases obtenues à l'étape (b);
(d) déterminer la structure d'au moins une portion du complexe en se basant sur ladite carte de densité des électrons.

16. Méthode de la revendication 15, où les coordonnées de structure utilisées à l'étape (a) sont les mêmes que ou s'écartent d'au moins d'environ 2 Å de celles décrites au Tableau 1.

17. Méthode pour évaluer l'aptitude d'une entité chimique à s'associer à un ligand de CD40 ou CD40, un fragment de CD40 ou d'un ligand de CD40 ou un complexe comprenant un ligand de CD40, CD40 ou leurs homologues, ladite méthode comprenant les étapes de:
(a) employer des moyens de calcul ou expérimentaux pour accomplir une opération d'ajustement entre l'entité chimique et ledit ligand de CD40 ou CD40, fragment ou complexe de celui-ci, pour ainsi obtenir des données en rapport avec ladite association, et
(b) analyser les données obtenues à l'étape (a) pour déterminer les caractéristiques de l'association entre l'entité chimique et ledit ligand de CD40 ou CD40, fragment ou complexe.

18. Méthode de la revendication 17, comprenant de plus l'identification, la caractérisation ou la désignation d'une entité chimique ayant une association souhaitée avec un ligand de CD40 ou son fragment, en utilisant les coordonnées de structure d'un cristal du ligand de CD40 de l'une quelconque des revendications 11 à 14.

19. Méthode de la revendication 18, comprenant de plus l'étape d'optimiser les caractéristiques de liaison de l'entité chimique identifiée, caractérisée ou désignée.

20. Méthode de la revendication 15, comprenant de plus l'étape de déterminer l'orientation des ligands dans un site de liaison du ligand de CD40.

21. Dérivé d'atome lourd d'une forme cristallisée d'un ligand de CD40.

22. Dérivé d'atome lourd du cristal de l'une quelconque des revendications 11 à 14 .

23. Utilisation des coordonnées de structure du ligand de CD40, ou ses portions, pour résoudre une forme cristalline d'un homologue mutant ou co-complexe d'un ligand de CD40 ou son fragment par remplacement moléculaire.

24. Utilisation d'un cristal du ligand de CD40 de l'une quelconque des revendications 11 à 14, ou ses coordonnées de structure, pour évaluer par calcul ou expérimentalement une entité chimique pour obtenir une information concernant son association avec le site de liaison du ligand de CD40.

25. Utilisation de la revendication 24, où le cristal a les coordonnées de structure décrites au Tableau 1.

26. Utilisation de la revendication 24, où ledit cristal est le même que le cristal du ligand de CD40 décrit par les coordonnées du Tableau 1 ou par les coordonnées s'écartant d'au moins d'environ 2Å des coordonnées du Tableau 1.

27. Utilisation d'un cristal d'un ligand de CD40 de l'une quelconque des revendications 11 à 14 pour déterminer les interactions de liaison entre une entité chimique et un ligand de CD40.

28. Support de stockage de données lisible à la machine comprenant un matériau de stockage de données encodé par des données lisibles en machine qui, quand il est lu par une machine appropriée, est capable de présenter une représentation tridimensionnelle d'un cristal d'une molécule ou d'un complexe moléculaire comprenant un fragment de CD40L ayant un site de liaison comprenant les acides aminés Lys143, Arg203, Arg207 et Tyr145.

29. Méthode pour la production d'une composition pharmaceutique comprenant la méthode de l'une quelconque des revendications 17 à 20 et en formulant par ailleurs l'entité chimique identifiée à la dernière étape de la méthode de l'une quelconque des revendications 17 à 20 sous une forme pharmaceutiquement acceptable.
